# EUROPEAN PATENT APPLICATION

(11) **EP 2 166 066 A1**
(43) Date of publication of application: **24.03.2010**
(21) Application number: 08752528.3
(22) Date of filing: 09.05.2008
(51) Int. Cl.: C10M 129/72, C07D 209/48, C10M 133/04, C10M 133/16, C10M 135/20, C10N 30/06

(54) **ADDITIVE FOR OILS AND LUBRICANT CONTAINING THE SAME**

(30) Priority: 09.05.2007 JP 2007124620
(71) Applicant: Kyowa Hakko Chemical Co., Ltd., Tokyo 103-0022 (JP)
(72) Inventor: HIYOSHI, Satoshi, Yokkaichi-shi Mie 510-8502 (JP); KISHI, Junya, Yokkaichi-shi Mie 510-8502 (JP); NAKAYAMA, Shingo, Yokkaichi-shi Mie 510-8502 (JP); OHARA, Suguru, Yokkaichi-shi Mie 510-8502 (JP); ISOGAI, Yukihiro, Chuo-ku Tokyo 103-0022 (JP)
(74) Representative: Dossmann, Gérard
(86) International application number: PCT/JP2008/058645
(87) International publication number: WO 2008/140045

(57) **Abstract**

An additive for oils that is capable of imparting oils such as lubricant base oils or fuel oils with superior wear resistance properties or superior friction resistance properties and the like are provided. The additive for oils comprising a compound represented by formula (I):

[Chemical Formula 1] **(A)ₘ-W-(B)ₙ** (I)

wherein m represents an integer of 0 to 4, n represents an integer of 2 to 6, A represents hydroxy or amino, W represents hydrocarbon or the like, and B represents formula (II): 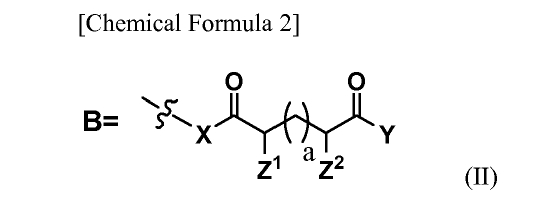 wherein a represents 0 or 1, X represents an oxygen atom or NH, Y represents OR¹ or NHR² (wherein R¹ and R² each represent alkyl optionally having one or more substituents or the like), and Z¹ and Z² each represent a hydrogen atom, NR³R⁴ (wherein R³ and R⁴ each represent alkyl optionally having one or more substituents or the like) or the like, and the like are provided.

## Description

### BACKGROUND OF THE INVENTION

### Field of the Invention

The present invention relates to an additive for oils comprising a compound that imparts excellent wear resistance properties or excellent friction resistance properties to oils such as lubricant base oils or fuel oils.
Priority is claimed on Japanese Patent Application No. 2007-124620, filed May 9, 2007, the content of which is incorporated herein by reference.

### Description of the Related Art

Lubricants generally contain a lubricant base oil and lubricant additives. Because metal components and phosphorus components have an adverse impact on the environment, lubricant additives containing no metal fraction or phosphorus fraction are in demand (see Non-Patent Documents 1 and 2).

Lubricants are required to exhibit a variety of properties, including wear resistance and friction resistance properties. Various additives for imparting lubricant base oils with wear resistance properties or friction resistance properties have been investigated (see Patent Document 1 and the like).
Volatile rust prevention oils containing an alkyl ester of an amino acid such as glutamic acid or aspartic acid and an oil are already known (see Patent Document 2).
Moreover, compounds such as the dimethyl ester of 2-phthalimidopentanedioic acid are known to be useful as raw materials for thalidomide (see Non-Patent Document 3).
[Patent Document 1]
   Japanese Patent (Granted) Publication No. 2,563,295
[Patent Document 2]
   Japanese Unexamined Patent Application, First Publication No. Sho 56-26995
[Non-Patent Document 1] "Journal of Economic Maintenance Tribology", July 2005 edition, page 7
[Non-Patent Document 2] "The Tribology", December 2005 edition, page 36
[Non-Patent Document 3] Organic Process Research & Development, vol. 9, 2005, page 853

### SUMMARY OF THE INVENTION

An object of the present invention is to provide an additive for oils comprising a compound that imparts oils such as lubricant base oils or fuel oils with superior wear resistance properties or superior friction resistance properties.

The present invention provides the aspects (1) to (29) described below. (1) An additive for oils, comprising a compound represented by formula (I),

[Chemical Formula 1] **(A)ₘ-W-(B)ₙ** (I)

wherein
m represents an integer of 0 to 4,
n represents an integer of 2 to 6,
m+n represents an integer of 2 to 6,
W is a group of valency (m+n) generated by removing (m+n) hydrogen atoms on carbon atoms from a compound selected from the group consisting of hydrocarbons of 2 to 20 carbon atoms, ethers of 4 to 20 carbon atoms, amines of 3 to 20 carbon atoms, sulfides of 4 to 20 carbon atoms and disulfides of 4 to 20 carbon atoms,
A represents hydroxy or amino,
when m is 2 or greater, As may be identical or different, and
Bs may be identical or different, and
B represents formula (II),

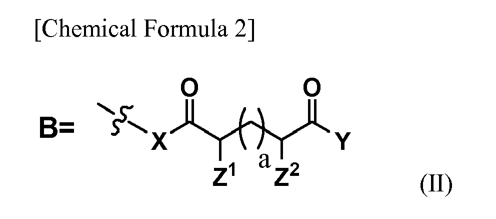

{wherein
"a"s are identical or different, and each represents 0 or 1,
Xs are identical or different, and each represents an oxygen atom or NH,
Ys are identical or different, and each represents OR¹ or NHR² (wherein R¹ and R² are identical or different, and each represents alkyl optionally having one or more substituents, alkenyl optionally having one or more substituents, aryl optionally having one or more substituents, aralkyl optionally having one or more substituents, cycloalkyl optionally having one or more substituents or cycloalkenyl optionally having one or more substituents),
Z¹s may be identical or different,
Z²s may be identical or different, and
within a single B, one of Z¹ and Z² represents a hydrogen atom, and another represents formula (III),

[Chemical Formula 3] **-NR³R⁴** (III)

[wherein R³ and R⁴ are identical or different, and each represents a hydrogen atom, alkyl optionally having one or more substituents, alkenyl optionally having one or more substituents, aryl optionally having one or more substituents, aralkyl optionally having one or more substituents, cycloalkyl optionally having one or more substituents, cycloalkenyl optionally having one or more substituents, alkanoyl optionally having one or more substituents, alkenoyl optionally having one or more substituents, aroyl optionally having one or more substituents, cycloalkylcarbonyl optionally having one or more substituents, alkyloxycarbonyl optionally having one or more substituents, alkenyloxycarbonyl optionally having one or more substituents, aryloxycarbonyl optionally having one or more substituents or cycloalkyloxycarbonyl optionally having one or more substituents, or R³ and R⁴ form a nitrogen-containing heterocyclic group optionally having one or more substituents in combination with an adjacent nitrogen atom thereto],
formula (IV),

[Chemical Formula 4] **-N=CR⁵R⁶** (IV)

[wherein R⁵ and R⁶ are identical or different, and each represents a hydrogen atom, alkyl optionally having one or more substituents or alkenyl optionally having one or more substituents, or R⁵ and R⁶ form cycloalkylidene optionally having one or more substituents in combination with an adjacent carbon atom thereto], or
formula (V),

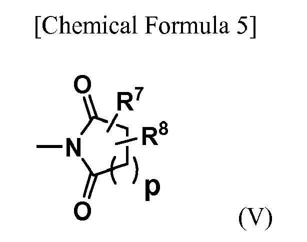

[wherein p represents an integer of 1 to 3, R⁷ and R⁸ are identical or different, and each represents a hydrogen atom, alkyl optionally having one or more substituents, alkenyl optionally having one or more substituents, aryl optionally having one or more substituents, aralkyl optionally having one or more substituents, cycloalkenyl optionally having one or more substituents, alkanoyl optionally having one or more substituents, alkenoyl optionally having one or more substituents, aroyl optionally having one or more substituents or cycloalkylcarbonyl optionally having one or more substituents, or R⁷ and R⁸ form, in combination with two carbon atoms adjacent thereto, cycloalkane optionally having one or more substituents or an aromatic ring optionally having one or more substituents]}.
(2) The additive for oils according to (1), wherein "a"s are identical, and each is 1.
(3) The additive for oils according to (1) or (2), wherein n is an integer of 2 to 4 and m is an integer of 0 to 2, m+n is an integer of 2 to 4, W is a group of valency (m+n) generated by removing (m+n) hydrogen atoms on carbon atoms from hydrocarbon, and said hydrocarbon is alkane of 2 to 10 carbon atoms.
(4) The additive for oils according to (1) or (2), wherein n is 2, m is 0, and W is formula (VI),

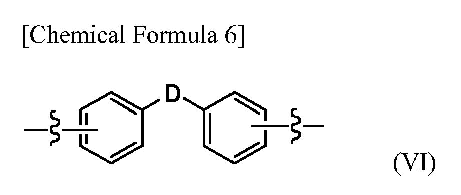

wherein D represents an oxygen atom, a sulfur atom, methylene or dimethylmethylene. (5) The additive for oils according to (1) or (2), wherein n is 2, m is 0, and W is formula (VII),

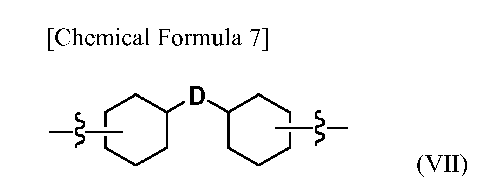

wherein D represents an oxygen atom, a sulfur atom, methylene or dimethylmethylene. (6) The additive for oils according to (1) or (2), wherein n is 2, m is 0, and W is formula (VIII),

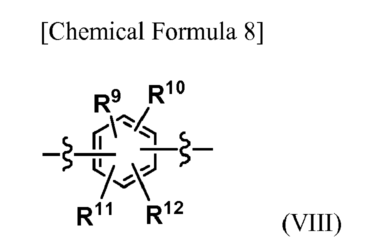

wherein R⁹, R¹⁰, R¹¹ and R¹² are identical or different, and each represents a hydrogen atom, methyl or ethyl.
(7) The additive for oils according to (1) or (2), wherein n is 2, m is 0, and W is formula (IX),

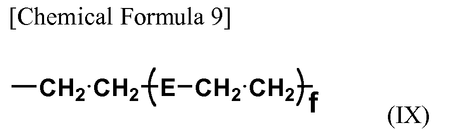

wherein f represents an integer of 1 to 4, and E represents an oxygen atom or formula (X),

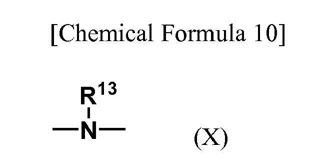

[wherein R¹³ represents a hydrogen atom, methyl or ethyl].
(8) The additive for oils according to (1) or (2), wherein n is 2, m is 0, and W is formula (XI),

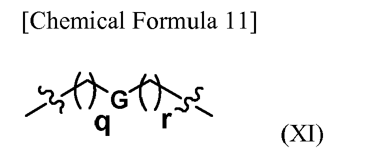

wherein G represents -S- or -S-S-, and q and r are identical or different and each represents 2 or 3.
(9) The additive for oils according to (8), wherein q is 2 and r is 2.
(10) The additive for oils according to (1) or (2), wherein n is 2 or 3, and m is 0 or 1, m+n is 3, and W is formula (XII).

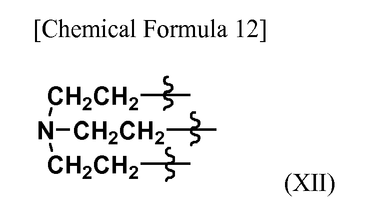

(11) The additive for oils according to (1) or (2), wherein n is 2 or 3, m is 0 or 1, m+n is 3, and W is formula (XIII).

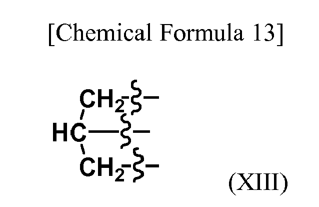

(12) The additive for oils according to (1) or (2), wherein n is 2 or 3, m is 0 or 1, m+n is 3, and W is formula (XIV),

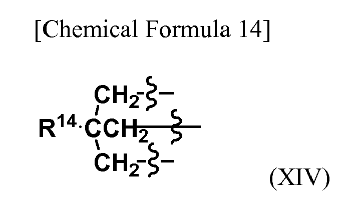

wherein R¹⁴ represents a hydrogen atom, alkyl of 1 to 6 carbon atoms, or alkenyl of 1 to 6 carbon atoms.
(13) The additive for oils according to (12), wherein R¹⁴ is ethyl.
(14) The additive for oils according to (1) or (2), wherein n is 2 or 3, m is 0 or 1, m+n is 3, and W is formula (XV).

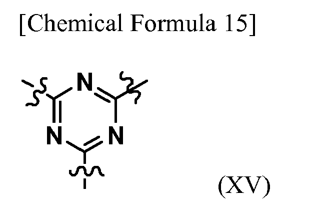

(15) The additive for oils according to (1) or (2), wherein n is an integer of 2 to 4, m is an integer of 0 to 2, m+n is 4, and W is formula (XVI).

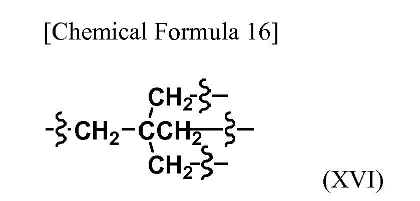

(16) The additive for oils according to (1) or (2), wherein n is an integer of 2 to 6, m is an integer of 0 to 4, m+n is 6, and W is formula (XVII).

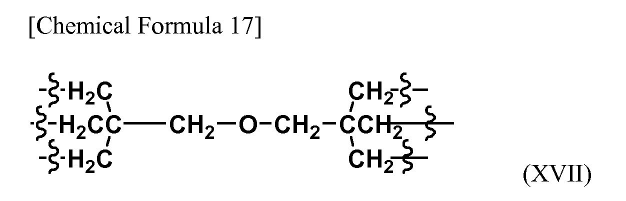

(17) The additive for oils according to any one of (1) to (16), wherein within a single B, one of Z¹ and Z² is a hydrogen atom, and another is formula (III).
(18) The additive for oils according to any one of (1) to (16), wherein within a single B, one of Z¹ and Z² is a hydrogen atom, and another is formula (V).
(19) The additive for oils according to (18), wherein formula (V) is phthalimido.
(20) The additive for oils according to any one of (1) to (19), wherein Xs are identical, and each is an oxygen atom.
(21) The additive for oils according to any one of (1) to (19), wherein Xs are identical, and each is NH.
(22) The additive for oils according to any one of (3) to (21), wherein Ys are identical, and each is OR¹ (wherein R¹ is as defined above).
(23) The additive for oils according to (22), wherein R¹ is alkyl optionally having one or more substituents or alkenyl optionally having one or more substituents.
(24) The additive for oils according to any one of (1) to (3) and (10) to (23), wherein m is 0.
(25) A lubricant, comprising the additive for oils according to any one of (1) to (24), and a lubricant base oil.
(26) The lubricant according to (25), wherein the lubricant base oil is at least one material selected from the group consisting of mineral oils, poly-α-olefins, fatty acid esters, polyalkylene glycols, phosphates, silicones, silicates, polyphenyl ethers, alkylbenzenes, synthetic naphthenes, gas-to-liquid (GTL) products, and vegetable oils.
(27) An ester compound represented by formula (Ia):

[Chemical Formula 18] **(HO)ₘₐ-W^{a}-(B^{a})ₙₐ** (Ia)

wherein:
ma represents an integer of 0 to 2,
na represents an integer of 2 to 4,
ma+na represents an integer of 2 to 4,
W^{a} is a group of valency (ma+na) generated by removing (ma+na) hydrogen atoms on carbon atoms from alkane of 2 to 10 carbon atoms, and
B^{a}s may be identical or different,
B^{a} represents a group represented by formula (IIa):

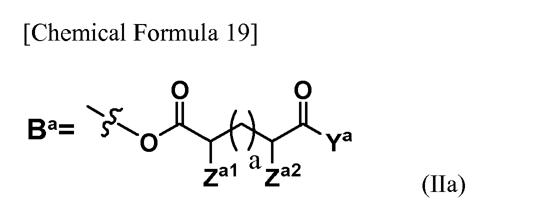

[wherein:
"a"s are identical or different, and each represents 0 or 1,
Y^{a}s are identical or different, and each represents -OR^{1a} (wherein R^{1a} represents alkyl of 1 to 20 carbon atoms optionally having one or more substituents, or alkenyl of 1 to 20 carbon atoms optionally having one or more substituents),
Z^{a1}s may be identical or different,
Z^{a2}s may be identical or different, and
within a single B^{a}, one of Z^{a1} and Z^{a2} represents a hydrogen atom, and the other represents phthalimido].
(28) The ester compound according to (27), wherein ma is 0.
(29) The ester compound according to (27) or (28), wherein R^{1a} is alkyl of 12 to 20 carbon atoms optionally having one or more substituents, or alkenyl of 12 to 20 carbon atoms optionally having one or more substituents.

The present invention is able to provide an additive for oils comprising a compound that is capable of imparting superior wear resistance properties or superior friction resistance properties to oils such as lubricant base oils or fuel oils.

### DETAILED DESCRIPTION OF THE INVENTION

The additive for oils of the present invention is added to an oil such as a lubricant base oil or a fuel oil or the like, thereby imparting the oil such as a lubricant base oil or fuel oil with wear resistance properties or friction resistance properties, and comprises a compound represented by formula (I). In the following description, this compound may be referred to as "compound (I)".

Examples of the hydrocarbons of 2 to 20 carbon atoms include alkanes, alkenes, cycloalkanes, cycloalkenes, aromatic hydrocarbons, alkanes containing aryls, alkanes containing cycloalkyl(s), and the like. Of these, alkanes are preferred.

The alkane is preferably a compound of 2 to 10 carbon atoms, and more preferably 2 to 6 carbon atoms. Specific examples of the alkane include ethane, propane, butane, pentane, hexane, heptane, octane, nonane, decane, undecane, dodecane, tridecane, tetradecane, hexadecane, octadecane, isobutane, isopentane, neopentane, 2,2-dimethylbutane, and the like.
The alkene is preferably a compound of 2 to 10 carbon atoms. Specific examples of the alkene include ethylene, propylene, 3-butene, 2-butene, 1-butene, 4-pentene, 3-pentene, 2-pentene, 1-pentene, octadecene, octadecadiene, 2-methyl-1-propene, 2-methyl-2-butene, and the like.

The cycloalkane is preferably a compound of 3 to 8 carbon atoms. Specific examples of the cycloalkane include cyclopropane, cyclobutane, cyclopentane, cyclohexane, cycloheptane, cyclooctane, cyclononane, cyclodecane, cyclopentadecane, cycloicosane, and the like.

The cycloalkene is preferably a compound of 3 to 8 carbon atoms. Specific examples of the cycloalkene include cyclopropene, cyclobutene, cyclopentene, cyclohexene, cyclopentene, cyclooctene, cyclononene, cyclodecene, cyclopentadecene, cycloicosene, and the like.

The aromatic hydrocarbon is preferably a compound of 6 to 10 carbon atoms. Specific examples of the aromatic hydrocarbon include benzene, naphthalene, anthracene, and the like.

The alkane containing aryl(s) is preferably a compound of 7 to 15 carbon atoms. Specific examples of the alkane containing aryl(s) include toluene, phenylethane, 3-phenylpropane, methylnaphthalene, diphenylmethane, 2,2-diphenylpropane, and the like.

The alkane containing cycloalkyl(s) is preferably a compound of 7 to 15 carbon atoms. Specific examples of the alkane containing cycloalkyl(s) include dicyclopropylmethane, dicyclopentylmethane, dicyclohexylmethane, and 2,2-dicyclohexylpropane, and the like.

Examples of the ethers of 4 to 20 carbon atoms include dialkyl ethers of 4 to 20 carbon atoms, dicycloalkyl ethers of 6 to 20 carbon atoms, diaryl ethers of 12 to 20 carbon atoms, and linear polyethers of 6 to 10 carbon atoms, and the like.
The two alkyls in the dialkyl ether of 4 to 20 carbon atoms may be identical or different, and are each selected from the groups within the following definition of "alkyl" so that the number of carbon atoms within the dialkyl ether falls within the specified range from 4 to 20. Specific examples of the alkyls include ethyl, propyl, butyl, neopentyl, and the like.
The two cycloalkyls in the dicycloalkyl ether of 6 to 20 carbon atoms may be identical or different, and are each selected from the groups within the following definition of "cycloalkyl" so that the number of carbon atoms within the dicycloalkyl ether falls within the specified range from 6 to 20. Specific examples of the cycloalkyls include cyclohexyl, and the like.

The two aryls in the diaryl ether of 12 to 20 carbon atoms may be identical or different, and are each selected from the groups within the following definition of "aryl" so that the number of carbon atoms within the diaryl ether falls within the specified range from 12 to 20. Specific examples of the aryls include phenyl, naphthyl, and the like.
Examples of the linear polyethers of 6 to 10 carbon atoms include 3,6-dioxaoctane, 3,6,9-trioxaundecane, 3,6,9,12-tetraoxatetradecane, and the like.
Examples of the amines of 3 to 20 carbon atoms include trialkylamines of 6 to 20 carbon atoms, triphenylamine, cyclic amines of 3 to 10 carbon atoms, and linear polyamines of 4 to 10 carbon atoms and N-alkylated compounds thereof, and the like.
The three alkyls in the trialkylamine of 6 to 20 carbon atoms are each selected from the groups within the following definition of "alkyl" so that the number of carbon atoms within the trialkylamine falls within the specified range from 6 to 20. Specific examples of the alkyls include ethyl, propyl, butyl, and the like.

Examples of the cyclic amines of 3 to 10 carbon atoms include pyridine, pyrazine, triazine, quinoline, acridine, phenazine, and the like.
Examples of the linear polyamines of 4 to 10 carbon atoms and N-alkylated compounds thereof include 3,6-diazaoctane, 3,6,9-triazaundecane, 3,6,9,12-tetraazatetradecane, and the N-methylated and N-ethylated products of these compounds, and the like.
Examples of the sulfides of 4 to 20 carbon atoms include dialkylsulfides of 4 to 20 carbon atoms, and the like.
Examples of the disulfides of 4 to 20 carbon atoms include dialkyldisulfides of 4 to 20 carbon atoms, and the like.

The two alkyls in the dialkyl sulfide of 4 to 20 carbon atoms, and the two alkyls in the dialkyl disulfide of 4 to 20 carbon atoms are as defined for the two alkyls in the aforementioned dialkyl ether of 4 to 20 carbon atoms, and specific examples thereof include ethyl, propyl, butyl, neopentyl, and the like.
Examples of the alkyl include linear or branched alkyls of 1 to 20 carbon atoms and the like. Specific examples of the linear alkyls of 1 to 20 carbon atoms include methyl, ethyl, propyl, butyl, pentyl, hexyl, heptyl, octyl, nonyl, decyl, undecyl, dodecyl, tridecyl, tetradecyl, hexadecyl, octadecyl, and the like. Specific examples of the branched alkyls of 3 to 20 carbon atoms include isobutyl, sec-butyl, tert-butyl, neopentyl, and the like.

Examples of the alkenyl include linear or branched alkenyls of 2 to 20 carbon atoms, and the like. Specific examples of the linear alkenyls of 2 to 20 carbon atoms include vinyl, allyl, 3-buten-1-yl, 2-buten-1-yl, 1-buten-1-yl, 4-penten-1-yl, 3-penten-1-yl, 2-penten-1-yl, 1-penten-1-yl, octadecenyl, octadecadienyl, and the like, and of these, octadecenyl is preferred. Specific examples of the branched alkenyls of 3 to 20 carbon atoms include isopropenyl, 2-methyl-1-propen-1-yl, and the like.

The cycloalkyl is preferably a group of 3 to 20 carbon atoms, and specific examples include cyclopropyl, cyclobutyl, cyclopentyl, cyclohexyl, cycloheptyl, cyclooctyl, cyclononyl, cyclodecyl, cyclopentadecyl, cycloicosyl, and the like.
The cycloalkenyl is preferably a group of 3 to 20 carbon atoms, and specific examples include cyclopropenyl, cyclobutenyl, cyclopentenyl, cyclohexenyl, cycloheptenyl, cyclooctenyl, cyclononeyl, cyclodecenyl, cyclopentadecenyl, cycloicosenyl, and the like.

In the alkanoyl, the alkyl portion is as defined above for the alkyl.
The alkanoyl is preferably a group of 2 to 21 carbon atoms, and specific examples include acetyl, propanoyl, butanoyl, pentanoyl, pivaloyl, hexanoyl, heptanoyl, octanoyl, nonanoyl, decanoyl, dodecanoyl, tridecanoyl, tetradecanoyl, hexadecanoyl, octadecanoyl, and the like.
In the alkenoyl, the alkenyl portion is as defined above for the alkenyl.
The alkenoyl is preferably a group of 3 to 21 carbon atoms, and specific examples include acryloyl, methacryloyl, octadecenoyl, octadecadienoyl, and the like.

The aryl is preferably a group of 6 to 20 carbon atoms, and specific examples include phenyl, biphenyl, naphthyl, and the like.
In the aralkyl, the alkyl portion is as defined above for the alkyl, and the aryl portion is as defined above for the aryl.
The aralkyl is preferably a group of 7 to 20 carbon atoms, and specific examples include benzyl, phenethyl, 3-phenylpropyl, naphthylmethyl, biphenylmethyl, and the like.
In the aroyl, the aryl portion is as defined above for the aryl.
The aroyl is preferably a group of 7 to 21 carbon atoms, and specific examples include benzoyl, naphthoyl, and the like.
In the cycloalkylcarbonyl, the cycloalkyl portion is as defined above for the cycloalkyl.

The cycloalkylcarbonyl is preferably a group of 4 to 21 carbon atoms, and specific examples include cyclopropylcarbonyl, cyclobutylcarbonyl, cyclopentylcarbonyl , cyclohexylcarbonyl, and the like.
In the alkyloxycarbonyl, the alkyl portion is as defined above for the alkyl.
The alkyloxycarbonyl is preferably a group of 2 to 21 carbon atoms, and specific examples include methoxycarbonyl, ethoxycarbonyl, propyloxycarbonyl, tert-butoxycarbonyl (BOC), and the like.
In the alkenyloxycarbonyl, the alkenyl portion is as defined above for the alkenyl.
The alkenyloxycarbonyl is preferably a group of 3 to 21 carbon atoms, and specific examples include allyloxycarbonyl, and the like.
In the cycloalkyloxycarbonyl, the cycloalkyl portion is as defined above for the cycloalkyl.

The cycloalkyloxycarbonyl is preferably a group of 4 to 21 carbon atoms, and specific examples include cyclopropyloxycarbonyl, cyclopentyloxycarbonyl, cyclohexyloxycarbonyl, and the like.
In the aryloxycarbonyl, the aryl portion is as defined above for the aryl.
The aryloxycarbonyl is preferably a group of 7 to 21 carbon atoms, and specific examples include phenyloxycarbonyl, naphthyloxycarbonyl, biphenyloxycarbonyl, and the like.
Examples of the aromatic ring include aromatic hydrocarbons of 6 to 20 carbon atoms, and specific examples include benzene, naphthalene, anthracene, naphthacene, pyrene, and the like.

Examples of the nitrogen-containing heterocyclic group include 5-membered or 6-membered monocyclic heterocyclic groups, and condensed bicyclic or tricyclic heterocyclic groups containing condensed 3- to 8-membered rings. These monocyclic heterocyclic groups and condensed polycyclic heterocyclic groups may include, besides the one nitrogen atom, an atom selected from among an oxygen atom, a sulfur atom and another nitrogen atom. Specific examples of the nitrogen-containing heterocyclic group include aziridinyl, azetidinyl, pyrrolidinyl, piperidino, azepanyl, 1,2,5,6-tetrahydropyridyl, imidazolidinyl, pyrazolidinyl, piperazinyl, homopiperazinyl, pyrazolinyl, oxazolidinyl, morpholino, thiazolidinyl, thiomorpholino, 2H-oxazolyl, 2H-thiazolyl, dihydroindolyl, dihydroisoindolyl, benzimidazolidinyl, dihydrobenzoxazolyl, dihydrobenzothiazolyl, tetrahydroquinolyl, tetrahydroisoquinolyl, tetrahydroquinoxalinyl, tetrahydroquinazolinyl, 2-pyrrolidinon-1-yl, 2-piperidinon-1-yl, and the like.

The cycloalkylidene is preferably a compound of 3 to 20 carbon atoms, and specific examples include cyclopropylidene, cyclobutylidene, cyclopentylidene, cyclohexylidene, cyclooctylidene, cyclopentadecylidene, and the like.
In the cycloalkane that optionally having one or more substituents, the cycloalkane is as defined above for the cycloalkane.

The substituent(s) of the alkyl optionally having one or more substituents,
the substituent(s) of the alkenyl optionally having one or more substituents,
the substituent(s) of the alkanoyl optionally having one or more substituents,
the substituent(s) of the alkenoyl optionally having one or more substituents,
the substituent(s) of the alkyloxycarbonyl optionally having one or more substituents, and
the substituent(s) of the alkenyloxycarbonyl optionally having one or more substituents
each represents 1 to 5 substituents that may be identical or different, wherein specific examples of the substituent(s) include alkoxy, alkylthio, alkyldithio, carbamoyl, azo, nitro, cyano, a halogen atom, and the like. The alkyl portion within the alkoxy, the alkylthio and the alkyldithio is as defined above for the alkyl. Specific examples of the halogen atom include fluorine, chlorine, bromine and iodine.

The substituent(s) of the aromatic ring optionally having one or more substituents,
the substituent(s) of the nitrogen-containing heterocyclic group optionally having one or more substituents,
the substituent(s) of the cycloalkane optionally having one or more substituents,
the substituent(s) of the aryl optionally having one or more substituents,
The substituent(s) of the aralkyl optionally having one or more substituents,
the substituent(s) of the cycloalkyl optionally having one or more substituents,
the substituent(s) of the cycloalkenyl optionally having one or more substituents, the substituent(s) of the aroyl optionally having one or more substituents,
the substituent(s) of the cycloalkylcarbonyl optionally having one or more substituents,
the substituent(s) of the aryloxycarbonyl optionally having one or more substituents,
the substituent(s) of the cycloalkyloxycarbonyl optionally having one or more substituents, and
the substituent(s) of the cycloalkylidene optionally having one or more substituents,
each represents 1 to 5 substituents that may be identical or different, wherein specific examples of the substituent(s) include alkyl, alkenyl, alkynyl, alkoxy, alkylthio, carbamoyl, azo, nitro, cyano, and a halogen atom, and the like. The alkyl, the alkenyl, the alkoxy, the alkylthio, the alkyldithio, and the halogen atom are as defined above.

The compound (I) described above may be used without modification as an additive for oils, but may also be converted to a salt or the like prior to use.
Examples of the salt include acid addition salts, amino acid addition salts, and the like.

Examples of the acid addition salts include organic acid salts, inorganic acid salts, and the like. Specific examples of organic acid salts include carboxylates, sulfonates, and the like, preferred examples include formate, acetate, trifluoroacetate, propionate, methanesulfonate, p-toluenesulfonate and trifluoromethanesulfonate salts, and the like, and of these, methanesulfonate salts are particularly desirable. Specific examples of inorganic acid salts include hydrochloride, hydrobromide, hydroiodide, nitrate, sulfate, carbonate and borate salts, and the like (but excluding phosphate salts), and of these, borate salts, and the like are particularly desirable.
Specific examples of amino acid addition salts include addition salts of lysine, glycine, phenylalanine, aspartic acid, glutamic acid, and the like.

When it is desirable that the compound (I) is obtained as a salt, the salt may simply be purified in those cases where the compound is obtained in the form of a salt, or in those cases where the compound is obtained in free form, the compound (I) may be dissolved or suspended in an appropriate solvent, and an acid or base added to isolate the salt, which may then be purified.
Further, the compound (I) or salt therefor may sometimes exist as an adduct with water or any of various solvents, and these adducts may also be used as the additive for oils of the present invention.

The compound (I) includes some compounds that may have stereoisomers such as geometric isomers, optical isomers and tautomers, but any of these isomers, including all possible isomeric forms and mixtures thereof, may be used as the additive for oils of the present invention.

Next is a description of the method for producing the compound (I), based on a series of examples.
For example, the compound (I) can be produced in accordance with reaction 1.

### (Reaction 1)

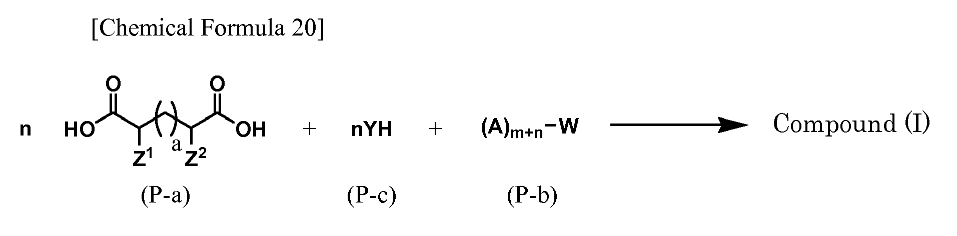

wherein m, n, a, A, W, Y, Z¹ and Z² are each as defined above.
The compound (I) can be produced, for example, by reacting compound (P-a), compound (P-b) and compound (P-c), in the presence of a catalyst, for 1 to 100 hours at a temperature of 100 to 200°C.
The compound (P-a) may be either obtained as a commercial product, or produced in accordance with a method such as those disclosed in Journal of American Chemical Society, 77, 1955, p. 2843, Chemistry Letters, 1984, p. 441, Japanese Unexamined Patent Application, First Publication No. Sho 63-2962 and Japanese Unexamined Patent Application, First Publication No. Sho 60-92250.

Among the various compounds (P-b), polyols containing 2 to 6 hydroxys may be either obtained as commercial products, or produced in accordance with methods such as those disclosed in U.S. Pat. No. 4,076,758 and Japanese Unexamined Patent Application, First Publication No. Sho 58-8027.
Among the various compounds (P-b), polyamines containing 2 to 6 aminos may be either obtained as commercial products, or produced in accordance with methods such as those disclosed in Japanese Unexamined Patent Application, First Publication No. Sho 54-62300 and Japanese Unexamined Patent Application, First Publication No. Hei 3-204840.
Among the various compounds (P-b), aminoalcohols containing a total of 2 to 6 amino(s) and hydroxy(s) may be either obtained as commercial products, or produced in accordance with methods such as those disclosed in Japanese Laid-Open Patent Application No. 2000-344695 and Japanese Laid-Open Patent Application No. 2001-89403.

Among the various compounds (P-c), alcohols in which YH is R¹OH (wherein R¹ is as defined above) may be either obtained as commercial products, or produced in accordance with methods such as those disclosed in Japanese Laid-Open Patent Application No. 2000-344695 and Japanese Laid-Open Patent Application No. 2001-89403.
Among the various compounds (P-c), primary amines in which YH is R²NH₂ (wherein R² is as defined above) may be either obtained as commercial products, or produced in accordance with methods such as those disclosed in U.S. Pat. No. 4,409,399 and Japanese Examined Patent Application, Second Publication No. Sho 38-21353.

When performing reaction 1, relative to the total number of moles of hydroxy and amino contained within the compound (P-b), the amount used of the compound (P-a) is preferably within a range from 0.8 to 3 equivalents, and the amount used of the compound (P-c) is preferably within a range from 0.8 to 3 equivalents.
Examples of the catalyst include Bronsted acid catalysts such as methanesulfonic acid, and tetrabutyl titanate ester.
Relative to the amount of the compound (P-c), the amount of the catalyst is preferably within a range from 0.01 to 20 equivalents, and is more preferably from 0.05 to 5 equivalents.
A solvent may be used during the reaction, and examples of solvents that may be used include hydrocarbons such as decane, tetradecane, toluene and xylene, ethers such as dibutyl ether, methoxybenzene and diphenyl ether, halogenated solvents such as dichloroethane, chlorobenzene and dichlorobenzene, amides such as N,N-dimethylformamide and N,N-dimethylacetamide, and dimethylsulfoxide.
In those cases where m+n is 3 or greater, reaction 1 may sometimes yield a mixture of two or more compounds (I) having different values for n. For example, when glutamic acid, pentaerythritol and stearyl alcohol are reacted in accordance with reaction 1, a mixture is sometimes obtained that may include two or more compounds having different values for n, such as a condensate of 4 molecules of the monoester of glutamic acid and stearyl alcohol and 1 molecule of pentaerythritol (n=4), a condensate of 3 molecules of the monoester and 1 molecule of pentaerythritol (n=3), and a condensate of 2 molecules of the monoester and 1 molecule of pentaerythritol (n=2). In the following description, in those cases where the two or more compounds (I) within the mixture are each an ester, the mixture may sometimes be referred to as a "mixed ester".

Following reaction, if required, the compound (I) may be purified using the types of methods typically employed in organic synthetic chemistry (such as the various chromatographic methods, recrystallization methods and distillation methods). In those cases where reaction 1 yields two or more compounds (I), the types of purification methods listed above may be used to separate the compounds, or the two or more compounds (I) may be used, without further modification, as the additive for oils.
Another method for producing the compound (I) is a method in which the compound (I) is produced via reactions 2 and 3. The compounds (P-a), (P-b) and (P-c) mentioned below are as defined above.

### (Reaction 2)

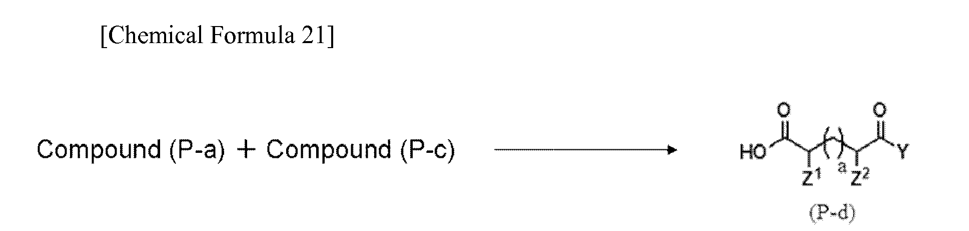

wherein a, Y, Z¹ and Z² are each as defined above.

### (Reaction 3)

[Chemical Formula 22] n Compound (P-d) + Compound (P-b) → Compound (I)

wherein n is as defined above.

### (Reaction 2)

The compound (P-a) and the compound (P-c) are reacted, in the presence of a condensation agent, for 1 to 100 hours at a temperature of 0 to 100°C, yielding a compound (P-d). The compound (P-d) is a compound produced by the condensation of 1 molecule of the compound (P-a) and 1 molecule of the compound (P-c).
The amount used of the compound (P-c) is preferably within a range from 0.8 to 3 equivalents, relative to the amount of the compound (P-a).

Examples of the condensation agent include dicyclohexylcarbodiimide, 1-ethyl-3-(N,N'-dimethylaminopropyl)carbodiimide, benzotriazol-1-yl-oxy-tris(dimethylamino)phosphonium salts, and the like.
Relative to the amount of the compound (P-c), the amount of the condensation agent is preferably within a range from 1 to 20 equivalents, and is more preferably from 1 to 5 equivalents.
A solvent may be used during the reaction, and examples of solvents that may be used include hydrocarbons such as hexane, decane, tetradecane, toluene and xylene, ethers such as diethyl ether, dibutyl ether, methoxybenzene and diphenyl ether, halogenated solvents such as methylene chloride, dichloroethane, chloroform, chlorobenzene and dichlorobenzene, amides such as N,N-dimethylformamide and N,N-dimethylacetamide, and dimethylsulfoxide.
Following the reaction, the compound (P-d) may be purified using the types of methods typically employed in organic synthetic chemistry (such as the various chromatographic methods, recrystallization methods and distillation methods), or the reaction mixture may be supplied, without further modification, to reaction 3.

### (Reaction 3)

The compound (P-d) obtained in reaction 2 and the compound (P-b) are reacted under the same conditions as those described for reaction 2, yielding the compound (I). The compound (I) is a compound obtained by the condensation of n molecules of the compound (P-d) and 1 molecule of the compound (P-b) [hereafter, the compound (I) may also be referred to as a condensate of the compound (P-d) and the compound (P-b)].
Relative to the total number of moles of hydroxy and amino contained within the compound (P-b), the amount used of the compound (P-d) is preferably within a range from 0.8 to 3 equivalents. In those cases where m+n is 3 or greater, reaction 3 may yield a mixture of two or more compounds (I) having different values for n.

Following reaction, if required, the compound (I) may be purified using the types of methods typically employed in organic synthetic chemistry (such as the various chromatographic methods, recrystallization methods and distillation methods). In those cases where reaction 3 yields two or more compounds (I), the types of purification methods listed above may be used to separate the compounds, or the two or more compounds (I) may be used, without further modification, as the additive for oils.
If required, protective groups may be introduced at the active groups such as the amino prior to the reaction 1 or 2, with these protective groups then being suitably eliminated.

The introduction and elimination of protective groups at active groups may be performed using conventional methods [such as the methods disclosed in "Protective Groups in Organic Synthesis", third edition, authored by T.W. Greene, published by John Wiley & Sons Inc. (1999)].
Specific examples of such protective groups include a benzyloxycarbonyl group, a p-methoxybenzyloxycarbonyl group, a tert-butoxycarbonyl group, a 9-fluorenylmethoxycarbonyl group, a 3-nitro-2-pyridinesulfenyl group, an acetyl group, and the like.

Furthermore, compounds included within the definition of compound (I) may be converted to other compounds included within the definition of compound (I). For example, a compound (I) in which Z¹ or Z² is amino can be converted to a compound (I)
in which Z¹ or Z² is alkanoylamino using a conventional method (such as that disclosed in Japanese Unexamined Patent Application, First Publication No. Sho 63-2962 or Organic Synthesis, 4, 1963, page 339). In this example, the alkanoyl portion within the alkanoylamino is as defined above for the alkanoyl.
The ester compound represented by compound (Ia) can be produced in a similar manner to the compound (I).
Specific examples of preferred forms of the compound (I) include (I-1) to (I-19) shown below.

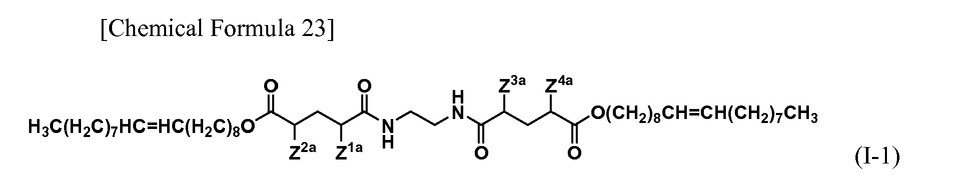

The compound (I-1) exists as a mixture of isomers.
The isomers are positional isomers generated by differences in Z^{1a} to Z^{4a}.
One of Z^{1a} and Z^{2a} is a hydrogen atom, and the other is amino.
One of Z^{3a} and Z^{4a} is a hydrogen atom, and the other is amino.

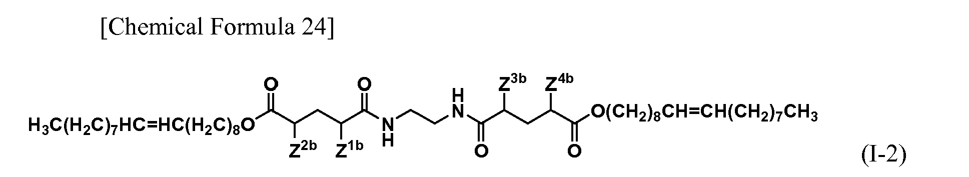

The compound (1-2) exists as a mixture of isomers.
The isomers are positional isomers generated by differences in Z^{1b} to Z^{4b}.
One of Z^{1b} and Z^{2b} is a hydrogen atom, and the other is acetylamino.
One of Z^{3b} and Z^{4b} is a hydrogen atom, and the other is acetylamino.

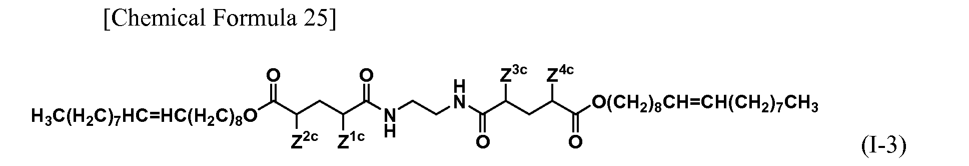

The compound (1-3) exists as a mixture of isomers.
The isomers are positional isomers generated by differences in Z^{1c} to Z^{4c}.
One of Z^{1c} and Z^{2c} is a hydrogen atom, and the other is (Z)-9-octadecenoylamino.
One of Z^{3c} and Z^{4c} is a hydrogen atom, and the other is (Z)-9-octadecenoylamino.

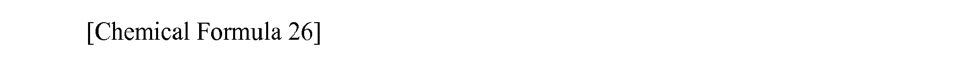

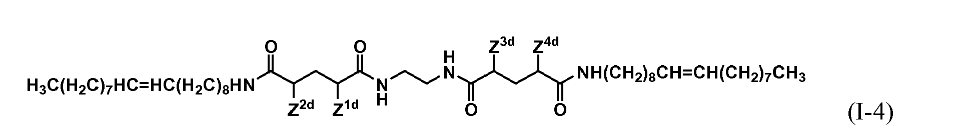

The compound (1-4) exists as a mixture of isomers.
The isomers are positional isomers generated by differences in Z^{1d} to Z^{4d}.
One of Z^{1d} and Z^{2d} is a hydrogen atom, and the other is acetylamino.
One of Z^{3d} and Z^{4d} is a hydrogen atom, and the other is acetylamino.

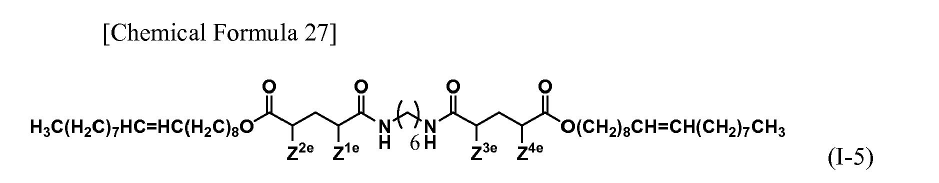

The compound (1-5) exists as a mixture of isomers.
The isomers are positional isomers generated by differences in Z^{1e} to Z^{4e}.
One of Z^{1e} and Z^{2e} is a hydrogen atom, and the other is amino.
One of Z^{3e} and Z^{4e} is a hydrogen atom, and the other is amino.

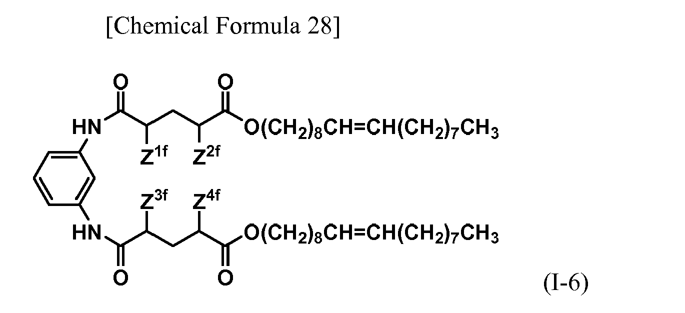

The compound (1-6) exists as a mixture of isomers.
The isomers are positional isomers generated by differences in Z^{1f} to Z^{4f}.
One of Z^{1f} and Z^{2f} is a hydrogen atom, and the other is amino.
One of Z^{3f} and Z^{4f} is a hydrogen atom, and the other is amino.

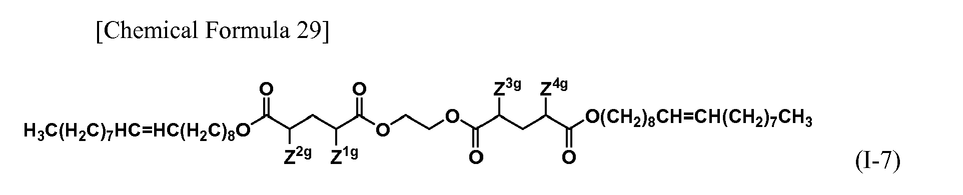

The compound (1-7) exists as a mixture of isomers.
The isomers are positional isomers generated by differences in Z^{1g} to Z^{4g}.
One of Z^{1g} and Z^{2g} is a hydrogen atom, and the other is amino.
One of Z^{3g} and Z^{4g} is a hydrogen atom, and the other is amino.

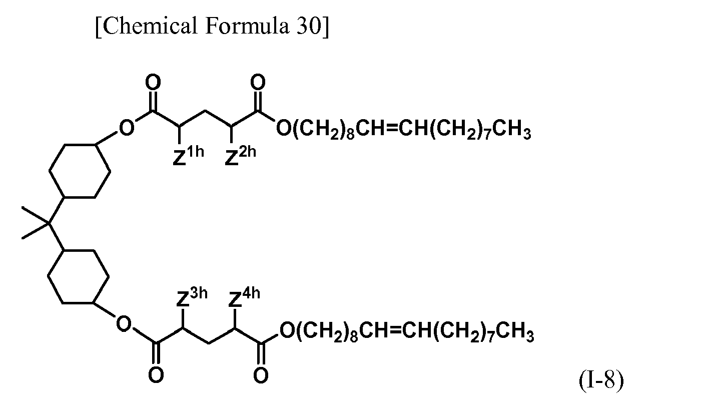

The compound (1-8) exists as a mixture of isomers.
The isomers are positional isomers generated by differences in Z^{1h} to Z^{4h}.
One of Z^{1h} and Z^{2h} is a hydrogen atom, and the other is amino.
One of Z^{3h} and Z^{4h} is a hydrogen atom, and the other is amino.

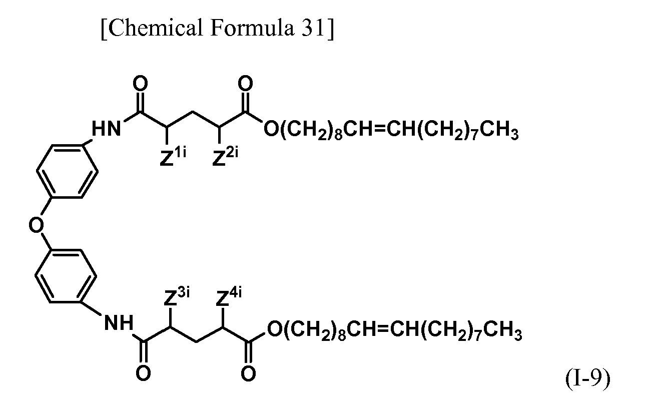

The compound (1-9) exists as a mixture of isomers.
The isomers are positional isomers generated by differences in Z¹ⁱ to Z⁴ⁱ.
One of Z¹ⁱ and Z²ⁱ is a hydrogen atom, and the other is amino.
One of Z³ⁱ and Z⁴ⁱ is a hydrogen atom, and the other is amino.

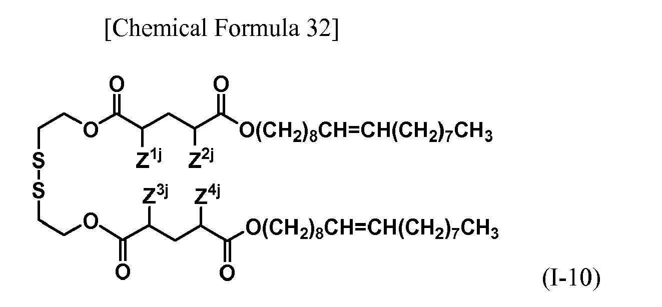

The compound (I-10) exists as a mixture of isomers.
The isomers are positional isomers generated by differences in Z^{1j} to Z^{4j}.
One of Z^{1j} and Z^{2j} is a hydrogen atom, and the other is amino.
One of Z^{3j} and Z^{4j} is a hydrogen atom, and the other is amino.

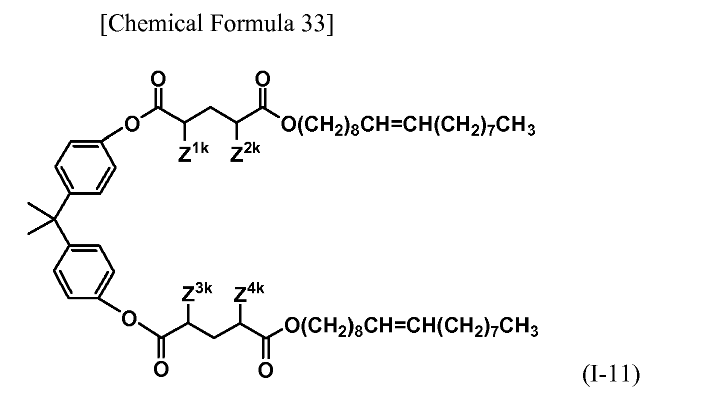

The compound (I-11) exists as a mixture of isomers.
The isomers are positional isomers generated by differences in Z^{1k} to Z^{4k}.
One of Z^{1k} and Z^{2k} is a hydrogen atom, and the other is amino.
One of Z^{3k} and Z^{4k} is a hydrogen atom, and the other is amino.

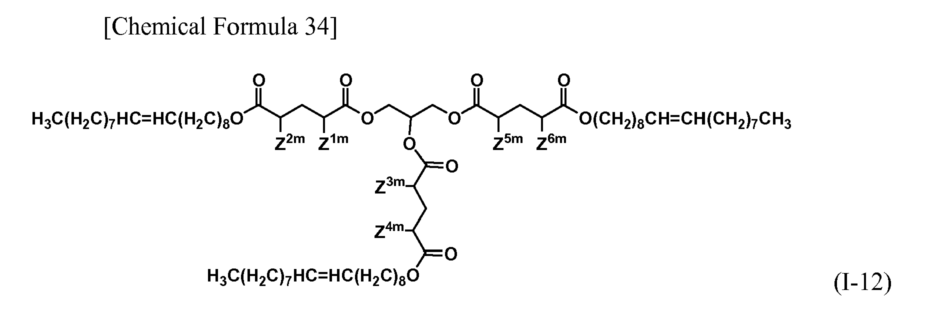

The compound (I-12) exists as a mixture of isomers.
The isomers are positional isomers generated by differences in Z^{1m} to Z^{6m}.
One of Z^{1m} and Z^{2m} is a hydrogen atom, and the other is amino. One of Z^{3m} and Z^{4m} is a hydrogen atom, and the other is amino.
One of Z^{5m} and Z^{6m} is a hydrogen atom, and the other is amino.

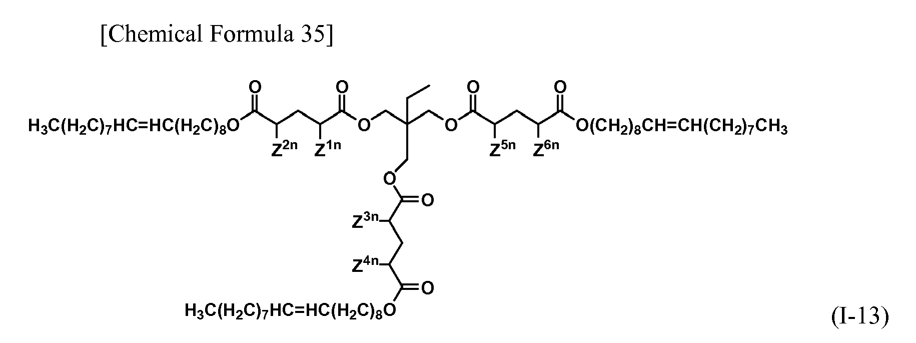

The compound (I-13) exists as a mixture of isomers.
The isomers are positional isomers generated by differences in Z¹ⁿ to Z⁶ⁿ.
One of Z¹ⁿ and Z²ⁿ is a hydrogen atom, and the other is amino.
One of Z³ⁿ and Z⁴ⁿ is a hydrogen atom, and the other is amino.
One of Z⁵ⁿ and Z⁶ⁿ is a hydrogen atom, and the other is amino.
Compound (I-14) is a mixed ester of 1 (or 5)-octadecyl hydrogen 2-aminopentanedioate and pentaerythritol. This mixed ester includes condensates of 1 molecule of pentaerythritol with 2, 3 and 4 molecules of 1 (or 5)-octadecyl hydrogen 2-aminopentanedioate.
Compound (I-15) is a mixed ester of 1 (or 5)-octadecyl hydrogen 2-aminopentanedioate and dipentaerythritol. This mixed ester includes condensates of 1 molecule of dipentaerythritol with 2, 3, 4, 5 and 6 molecules of 1 (or 5)-octadecyl hydrogen 2-aminopentanedioate.

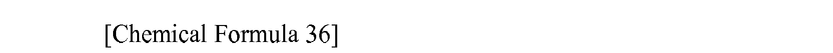

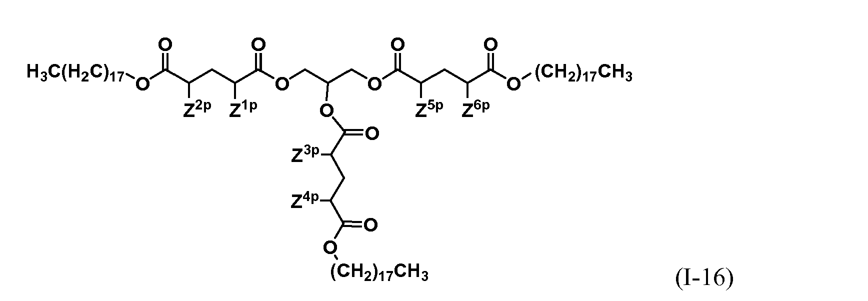

The compound (I-16) exists as a mixture of isomers.
The isomers are positional isomers generated by differences in Z^{1p} to Z^{6p}.
One of Z^{1p} and Z^{2p} is a hydrogen atom, and the other is phthalimido.
One of Z^{3p} and Z^{4p} is a hydrogen atom, and the other is phthalimido.
One of Z^{5P} and Z^{6p} is a hydrogen atom, and the other is phthalimido.

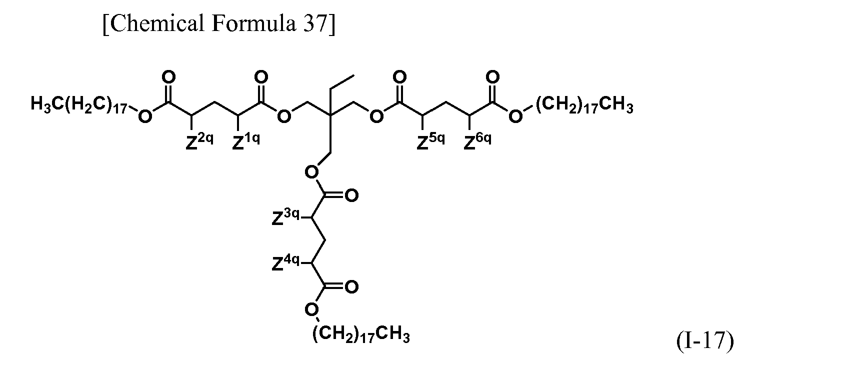

The compound (I-17) exists as a mixture of isomers.
The isomers are positional isomers generated by differences in Z^{1q} to Z^{6q}.
One of Z^{1q} and Z^{2q} is a hydrogen atom, and the other is phthalimido.
One of Z^{3q} and Z^{4q} is a hydrogen atom, and the other is phthalimido.
One of Z^{5q} and Z^{6q} is a hydrogen atom, and the other is phthalimido.

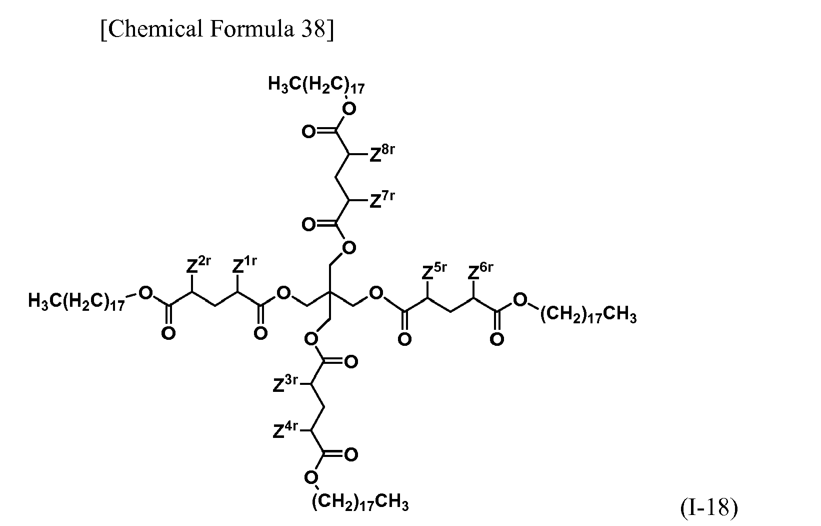

The compound (I-18) exists as a mixture of isomers.
The isomers are positional isomers generated by differences in Z^{1r} to Z^{8r}.
One of Z^{1r} and Z^{2r} is a hydrogen atom, and the other is phthalimido.
One of Z^{3r} and Z^{4r} is a hydrogen atom, and the other is phthalimido.
One of Z^{Sr} and Z^{6r} is a hydrogen atom, and the other is phthalimido.
One of Z^{7r} and Z^{8r} is a hydrogen atom, and the other is phthalimido.

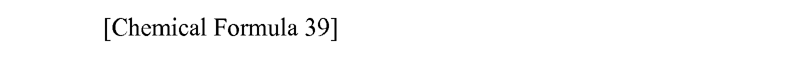

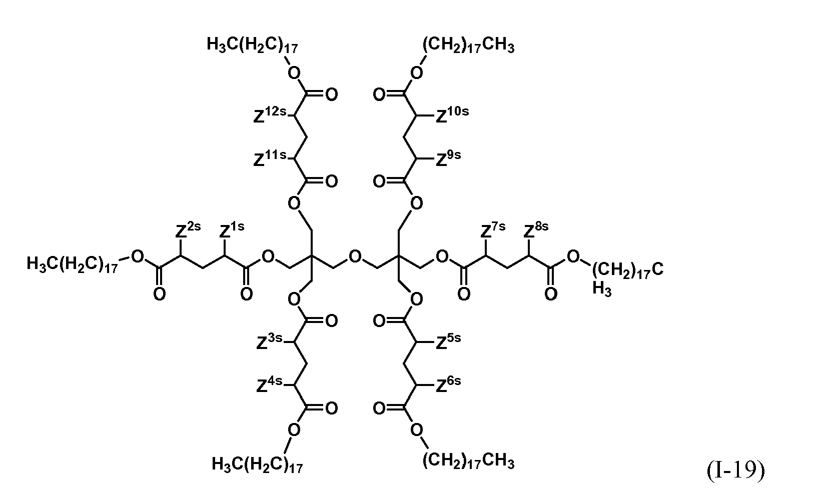

The compound (I-19) exists as a mixture of isomers.
The isomers are positional isomers generated by differences in Z^{1s} to Z^{12s}.
One of Z^{1s} and Z^{2s} is a hydrogen atom, and the other is phthalimido.
One of Z^{3s} and Z^{4s} is a hydrogen atom, and the other is phthalimido.
One of Z^{5s} and Z^{6s} is a hydrogen atom, and the other is phthalimido.
One of Z^{7s} and Z^{8s} is a hydrogen atom, and the other is phthalimido.
One of Z^{9s} and Z^{10s} is a hydrogen atom, and the other is phthalimido.
One of Z^{11s} and Z^{12s} is a hydrogen atom, and the other is phthalimido.
Among the various compounds (I), particularly preferred compounds include those for which, within formula (I),
m is 0,
n is 2 to 4,
W is a group of valency n generated by removing n hydrogen atoms on carbon atoms from alkane of 2 to 10 carbon atoms,
A is hydroxy,
Bs may be identical or different, and
within formula (II),
"a"s are identical, and each is 1,
Xs are identical, and each is an oxygen atom,
Ys may be identical or different, and each is -OR¹ (wherein the R¹ represents alkyl of 12 to 20 carbon atoms optionally having one or more substituents, or alkenyl of 12 to 20 carbon atoms optionally having one or more substituents),
Z¹s may be identical or different,
Z²s may be identical or different,
within a single B, one of Z¹ and Z² is a hydrogen atom, and the other is a group represented by formula (V), wherein formula (V) is phthalimido. The above alkyl optionally having one or more substituents and alkenyl optionally having one or more substituents are each as defined above.

A lubricant of the present invention comprises a lubricant base oil and an additive for oils comprising the compound (I). The amount of the compound (I) within the lubricant is preferably within a range from 0.001 to 300 mmol, more preferably from 0.01 to 200 mmol, and still more preferably from 0.1 to 100 mmol, per 1 kg of the lubricant. Provided the amount of the compound (I) is within this range, superior wear resistance properties or superior friction resistance properties can be imparted.

As the lubricant base oil, all manner of lubricant base oils, typified by natural base oils and synthetic base oils, may be used.
Examples of natural base oils include mineral oils, vegetable oils and animal oils.
Examples of mineral oils include paraffin base crude oils, intermediate base crude oils, naphthene base crude oils, and the like. Refined oils obtained by refining these types of oils by distillation or the like may also be used.
Examples of synthetic base oils include poly-α-olefins such as polybutene, polypropylene, and α-olefin oligomers of 8 to 14 carbon atoms; esters such as fatty acid monoesters, aromatic monoesters, fatty acid diesters, aromatic diesters, aliphatic polybasic acid esters, aromatic polybasic acid esters and polyol polyesters; as well as polyalkylene glycols, phosphates, silicones, silicates, polyphenyl ethers, alkylbenzenes, synthetic naphthenes, gas-to-liquid (GTL) products, fluorocarbons and ionic liquids.
Of these, preferred lubricant base oils include mineral oils, poly-α-olefins, fatty acid esters, polyalkylene glycols, phosphates, silicones, silicates, polyphenyl ethers, alkylbenzenes, synthetic naphthenes, gas-to-liquid (GTL) products, vegetable oils, and the like, and one or more of these oils is preferably used.

Besides the aforementioned lubricant base oil and the additive for oils comprising the compound (I), the lubricant of the present invention may also contain other typically employed additives as optional components, including detergent dispersants, antioxidants, wear reducers (wear resistance agents, seizure resistance agents, extreme pressure agents, and the like), friction modifiers, oiliness agents, rust prevention agents, vapor phase rust prevention agents, pour point depressants, viscosity index improvers, thickeners, preservatives, deformers, demulsifying agents, dyes and fragrances, and the like. The amount of each of these additives within the lubricant base oil is preferably within a range from 0.001 to 5% by weight.
The lubricant of the present invention can be used in engine oils, automatic transmission oils, continuously variable transmission oils, gear oils, power steeling oils, shock absorber oils, turbine oils, hydraulic oils, refrigeration oils, rolling oils, bearing oils, metalworking lubricants, sliding surface oils, greases, biolubricants, or the like.

The additive for oils of the present invention can be added not only to lubricant oils, but also to other oils such as fuel oils.
Examples of fuel oils include highly hydrorefined fuel oils, biodiesel fuels, and the like. The amount of the compound (I) within the fuel oil is preferably within a range from 0.00001 to 10% by mass, and more preferably from 0.00001 to 1% by mass. Provided the amount of the compound (I) is within this range, superior wear resistance properties or superior friction resistance properties can be imparted to the oil.
The fuel oil may also include all manner of other additives in addition to the additive for oils of the present invention.
Moreover, the additive for oils of the present invention can be used not only for addition to lubricant base oils, fuel oils or the like, but also as a solid lubricant. A solid lubricant describes a material that is added to, coated onto, or impregnated within a material such as a plastic or fiber or the like, or a product such as a recording medium, coating material, ink or film or the like for the purpose of reducing friction and wear.

More specifically, the additive for oils of the present invention can also be used in plastic gears, bearings, sliding members such as cams, thermosensitive recording media, magnetic recording media, transfer media, planographic printing plates, image receiving sheets, protective coating layers for toners, electronic photoreceptors or cleaning members which are used in electrophotographic components, as a protective coating layers for optical fibers, optic drop cables, polarizers and endoscopes or the like, optical films or the like.

### Examples

A more detailed description of the present invention is presented below based on a series of examples.
The measurement data reported in the examples were obtained using the measuring apparatuses and measuring techniques described below.
(1) Nuclear magnetic resonance spectra (¹H-NMR: conducted using tetramethylsilane as a standard): GSX-400 (400 MHz) (manufactured by JEOL Ltd.)
(2) Measurement of coefficient of kinetic friction (evaluation of friction resistance properties): Soda pendulum-type friction tester (manufactured by Shinko Engineering Co., Ltd.)
(3) Measurement of wear scar diameter (evaluation of wear resistance properties): Shell-type four-ball friction tester (manufactured by Takachiho Seiki Co., Ltd.)

### (Production example 1) 1 (or 5)-[(9Z)-9-octadecenyl] hydrogen 2-(N-tert-butoxycarbonylamino)pentanedioate (Compound A)

24.7 g of N-(tert-butoxycarbonyl)glutamic acid (manufactured by Kanto Chemical Co., Inc.), 21.5 g of (Z)-9-octadecen-1-ol (manufactured by Wako Pure Chemical Industries, Ltd.) and 12.2 g of 4-(dimethylamino)pyridine (manufactured by Wako Pure Chemical Industries, Ltd.) were dissolved in 400 mL of methylene chloride (manufactured by Wako Pure Chemical Industries, Ltd.), and following thorough stirring, 19.2 g of 1-ethyl-3-(N,N'-dimethylaminopropyl)carbodiimide (manufactured by Eiweiss Chemical Corporation) was added, and the resulting mixture was reacted at room temperature for 2 hours and then at 40°C for a further 7 hours. The reaction mixture was washed sequentially with 0.5 mol/L hydrochloric acid, a saturated aqueous solution of sodium bicarbonate, and a saturated aqueous saline solution. The organic layer was then dried over anhydrous magnesium sulfate, and the solvent was removed by distillation under reduced pressure at 50°C, yielding 39.3 g of the crude compound A.
This crude product was purified by silica gel column chromatography (developing solvent: n-hexane / ethyl acetate), yielding 18.3 g of the compound A (yield: 46%).

¹H-NMR (CDCl₃, δ ppm): 0.88 (t, 3H), 1.27 (br, 22H), 1.41 (br, 9H), 1.53 to 2.40 (m, 10H), 4.01 (br, 3H), 5.34 (m, 2H)
Elemental analysis result: C₂₈H₅₁NO₆
Calculated values (C: 67.57%, H: 10.33%, N: 2.81%)
Measured values (C: 67.49%, H: 10.40%, N: 2.82%)

### [Example 1] Condensate of 1 (or 5)-[(9Z)-9-octadecenyl] hydrogen 2-aminopentanedioate and ethylenediamine [Compound (I-1)]

4.9 g of 2-(N-tert-butoxycarbonylamino)pentanedioic acid (manufactured by Kanto Chemical Co., Inc.), 0.6 g of ethylenediamine (manufactured by Wako Pure Chemical Industries, Ltd.) and 2.4 g of 4-(dimethylamino)pyridine (manufactured by Wako Pure Chemical Industries, Ltd.) were dissolved in 50 mL of methylene chloride (manufactured by Wako Pure Chemical Industries, Ltd.), 4.2 g of 1-ethyl-3-(N,N'-dimethylaminopropyl)carbodiimide (manufactured by Eiweiss Chemical Corporation) was added, and the resulting mixture was reacted at room temperature for 2 hours and then at 40°C for a further 4 hours. To the resulting reaction mixture were added 5.4 g of (Z)-9-octadecen-1-ol (manufactured by Wako Pure Chemical Industries, Ltd.), 2.4 g of 4-(dimethylamino)pyridine and 50 mL of methylene chloride, and following thorough stirring, an additional 4.2 g of 1-ethyl-3-(N,N'-dimethylaminopropyl)carbodiimide was added, and the resulting mixture was reacted at room temperature for 2 hours and then at 40°C for a further 4 hours. Following reaction, the reaction mixture was washed sequentially with 0.5 mol/L hydrochloric acid, a saturated aqueous solution of sodium bicarbonate, and a saturated aqueous saline solution, the organic layer was dried over anhydrous magnesium sulfate, and the solvent was then removed by distillation under reduced pressure at 50°C, yielding 10.0 g of a condensate of 1 (or 5)-[(9Z)-9-octadecenyl] hydrogen 2-(N-tert-butoxycarbonylamino)pentanedioate and ethylenediamine.
To 10.0 g of the thus obtained condensate was added 250 mL of methylene chloride, and following stirring of the mixture under a nitrogen atmosphere until a uniform liquid was obtained, trifluoroacetic acid (manufactured by Wako Pure Chemical Industries, Ltd.) was added until thin layer chromatography confirmed that the raw material had been completely consumed (total amount of trifluoroacetic acid added: 52.5 g). Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 4.8 g of the compound (I-1) (yield: 58%).

Elemental analysis result: C₄₈H₉₀N₄O₆
Calculated values (C: 70.37%, H: 11.07%, N: 6.84%)
Measured values (C: 70.41%, H: 11.12%, N: 6.79%)

### (1) Measurement of coefficient of kinetic friction (evaluation of friction resistance properties)

The compound (I-1) was added to samples of a poly-α-olefin (DURASYN164, manufactured by INEOS Group Ltd., hereafter also referred to as "lubricant base oil A") or bis(3,5,5-trimethylhexyl) adipate (hereafter also referred to as "lubricant base oil B") in an amount equivalent to 10 mmol/kg, thus completing preparation of lubricant sample oils.
Subsequently, the coefficient of kinetic friction of each lubricant sample oil at 40°C, 80°C, 120°C and 150°C was measured using a Soda pendulum-type friction tester (manufactured by Shinko Engineering Co., Ltd.). The coefficient of kinetic friction was calculated from the initial amplitude of the pendulum, the amplitude upon oscillation, and the oscillation frequency. The results are shown in Table 1.

### (2) Measurement of wear scar diameter (evaluation of wear resistance properties)

Sample oils were prepared in the same manner as (1) above, and testing was conducted in accordance with the method prescribed in ASTM D4172 (loading: 40 kgf, revolution rate: 1,200 rpm, time: 60 minutes, temperature: 75°C), with the diameter of the wear scar being measured following completion of the testing. A shell-type four-ball friction tester (manufactured by Takachiho Seiki Co., Ltd.) was used as the test apparatus. The wear scar diameter was taken as the average of the wear scars in the vertical direction and the horizontal direction on the three fixed balls. The results are shown in Table 1.

### [Example 2] Condensate of 1 (or 5)-[(9Z)-9-octadecenyl] hydrogen 2-(N-acetylamino)pentanedioate and ethylenediamine [Compound (1-2)]

0.9 g of the compound (I-1) and 0.6 g of acetic anhydride (manufactured by Wako Pure Chemical Industries, Ltd.) were dissolved in 50 mL of methylene chloride, and the resulting solution was stirred under an atmosphere of nitrogen for 2 hours at room temperature. Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 0.7 g of the compound (1-2) (yield: 66%).

Elemental analysis result: C₅₂H₉₄N₄O₈
Calculated values (C: 69.14%, H: 10.49%, N: 6.20%)
Measured values (C: 69.28%, H: 10.55%, N: 6.23%)

With the exception of using the compound (1-2) as the additive for oils, sample oils were prepared in the same manner as example 1. Using the methods described above for example 1, the sample oil in which the lubricant base oil A was used was evaluated for friction resistance and wear resistance, whereas the sample oil in which the lubricant base oil B was used was evaluated for wear resistance. The results are shown in Table 1.

### [Example 3] Condensate of 1 (or 5)-[(9Z)-9-octadecenyl] hydrogen 2-{N-[(9Z)-9-octadecenoyl]amino}pentanedioate and ethylenediamine [Compound (1-3)]

0.9 g of the compound (I-1) and 0.4 g of triethylamine (manufactured by Wako Pure Chemical Industries, Ltd.) were dissolved in 50 mL of methylene chloride, 0.7 g of (Z)-9-octadecenoyl chloride (manufactured by Tokyo Chemical Industry Co., Ltd.) was added dropwise to the solution under an atmosphere of nitrogen, and the resulting mixture was stirred for 2 hours at room temperature. Following reaction, the reaction mixture was washed sequentially with distilled water, 0.5 mol/L hydrochloric acid, a saturated aqueous solution of sodium bicarbonate, and a saturated aqueous saline solution. The organic layer was dried over anhydrous magnesium sulfate, and the solvent was then removed by distillation under reduced pressure at 50°C, yielding 1.1 g of the compound (1-3) (yield: 68%).

Elemental analysis result: C₈₄H₁₅₄N₄O₈
Calculated values (C: 74.84%, H: 11.51%, N: 4.16%)
Measured values (C: 74.80%, H: 11.53%, N: 4.09%)

With the exception of using the compound (1-3) as the additive for oils, sample oils were prepared in the same manner as example 1, and then evaluated in the same manner as example 1. The results are shown in Table 1.

### [Example 4] Condensate of 2 (or 4)-acetylamino-4-[(9Z)-9-octadecenylcarbamoyl]butanoic acid and ethylenediamine [Compound (1-4)]

5.0 g of 2-(N-tert-butoxycarbonylamino)pentanedioic acid, 0.6 g of ethylenediamine and 2.5 g of 4-(dimethylamino)pyridine were dissolved in 50 mL of methylene chloride, 3.9 g of 1-ethyl-3-(N,N'-dimethylaminopropyl)carbodiimide was added, and the resulting mixture was reacted at room temperature for 2 hours and then at 40°C for a further 5 hours. To the resulting reaction mixture were added 5.4 g of (Z)-9-octadecenylamine (manufactured by Aldrich Co., Ltd.), 2.5 g of 4-(dimethylamino)pyridine and 50 mL of methylene chloride, and following thorough stirring, an additional 3.9 g of 1-ethyl-3-(N,N'-dimethylaminopropyl)carbodiimide was added, and the resulting mixture was reacted at room temperature for 1 hour and then at 40°C for a further 5 hours. Subsequently, the reaction mixture was washed sequentially with 0.5 mol/L hydrochloric acid, a saturated aqueous solution of sodium bicarbonate, and a saturated aqueous saline solution. The organic layer was dried over anhydrous magnesium sulfate, and the solvent was then removed by distillation under reduced pressure at 50°C, yielding 4.0 g of a condensate of 2 (or 4)-acetylamino-4-[(9Z)-9-octadecenyl]carbamoylbutanoic acid and ethylenediamine.
To 2.3 g of the thus obtained condensate was added 50 mL of methylene chloride, and following stirring of the mixture under a nitrogen atmosphere until a uniform liquid was obtained, trifluoroacetic acid was added until thin layer chromatography confirmed that the raw material had been completely consumed (total amount of trifluoroacetic acid added: 10.0 g). Following reaction, 2.8 g of acetic anhydride was added to the reaction mixture, and the resulting mixture was stirred at room temperature for 2 hours and then at 40°C for a further 7 hours under an atmosphere of nitrogen. Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 1.6 g of the compound (1-4) (yield: 31%).

Elemental analysis result: C₅₂H₉₆N₆O₆
Calculated values (C: 69.29%, H: 10.74%, N: 9.32%)
Measured values (C: 69.27%, H: 10.69%, N: 9.41%)

With the exception of using the compound (1-4) as the additive for oils, sample oils were prepared in the same manner as example 1, and then evaluated in the same manner as example 1. The results are shown in Table 2.

### [Example 5] Condensate of 1 (or 5)-[(9Z)-9-octadecenyl] hydrogen 2-aminopentanedioate and 1,6-hexanediamine [Compound (1-5)]

2.5 g of 2-(N-tert-butoxycarbonylamino)pentanedioic acid (manufactured by Kanto Chemical Co., Inc.), 0.6 g of 1,6-hexanediamine (manufactured by Tokyo Chemical Industry Co., Ltd.) and 1.2 g of 4-(dimethylamino)pyridine (manufactured by Wako Pure Chemical Industries, Ltd.) were dissolved in 25 mL of methylene chloride (manufactured by Wako Pure Chemical Industries, Ltd.), 2.1 g of 1-ethyl-3-(N,N'-dimethylaminopropyl)carbodiimide (manufactured by Eiweiss Chemical Corporation) was added, and the resulting mixture was reacted at room temperature for 1 hour and then at 40°C for a further 6 hours. To the resulting reaction mixture were added 2.7 g of (Z)-9-octadecen-1-ol (manufactured by Wako Pure Chemical Industries, Ltd.), 1.2 g of 4-(dimethylamino)pyridine and 25 mL of methylene chloride, and following thorough stirring, an additional 2.1 g of 1-ethyl-3-(N,N'-dimethylaminopropyl)carbodiimide was added, and the resulting mixture was reacted at room temperature for 1 hour and then at 40°C for a further 8 hours. Following reaction, the reaction mixture was washed sequentially with 0.5 mol/L hydrochloric acid, a saturated aqueous solution of sodium bicarbonate, and a saturated aqueous saline solution. The organic layer was dried over anhydrous magnesium sulfate, and the solvent was then removed by distillation under reduced pressure at 50°C, yielding 4.9 g of a condensate of 1 (or 5)-[(9Z)-9-octadecenyl] hydrogen 2-(N-tert-butoxycarbonylamino)pentanedioate and 1,6-hexanediamine.
To 2.9 g of the thus obtained condensate was added 40 mL of methylene chloride, and following stirring of the mixture under a nitrogen atmosphere until a uniform liquid was obtained, trifluoroacetic acid (manufactured by Wako Pure Chemical Industries, Ltd.) was added until thin layer chromatography confirmed that the raw material had been completely consumed (total amount of trifluoroacetic acid added: 12.0 g). Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 2.0 g of the compound (1-5) (yield: 75%).

Elemental analysis result: C₅₂H₉₈N₄O₆
Calculated values (C: 71.35%, H: 11.28%, N: 6.40%)
Measured values (C: 71.43%, H: 11.19%, N: 6.32%)

With the exception of using the compound (1-5) as the additive for oils, sample oils were prepared in the same manner as example 1, and then evaluated in the same manner as example 1. The results are shown in Table 2.

### [Example 6] Condensate of 1 (or 5)-[(9Z)-9-octadecenyl] hydrogen 2-aminopentanedioate and 1,3-phenylenediamine [Compound (1-6)]

2.5 g of 2-(N-tert-butoxycarbonylamino)pentanedioic acid (manufactured by Kanto Chemical Co., Inc.), 0.5 g of 1,3-phenylenediamine (manufactured by Tokyo Chemical Industry Co., Ltd.) and 1.2 g of 4-(dimethylamino)pyridine (manufactured by Wako Pure Chemical Industries, Ltd.) were dissolved in 25 mL of methylene chloride (manufactured by Wako Pure Chemical Industries, Ltd.), 2.1 g of 1-ethyl-3-(N,N'-dimethylaminopropyl)carbodiimide (manufactured by Eiweiss Chemical Corporation) was added, and the resulting mixture was reacted at room temperature for 1 hour and then at 40°C for a further 6 hours. To the resulting reaction mixture were added 2.7 g of (Z)-9-octadecen-1-ol (manufactured by Wako Pure Chemical Industries, Ltd.), 1.2 g of 4-(dimethylamino)pyridine and 25 mL of methylene chloride, and following thorough stirring, an additional 2.1 g of 1-ethyl-3-(N,N'-dimethylaminopropyl)carbodiimide was added, and the resulting mixture was reacted at room temperature for 1 hour and then at 40°C for a further 8 hours. Subsequently, the reaction mixture was washed sequentially with 0.5 mol/L hydrochloric acid, a saturated aqueous solution of sodium bicarbonate, and a saturated aqueous saline solution. The organic layer was dried over anhydrous magnesium sulfate, and the solvent was then removed by distillation under reduced pressure at 50°C, yielding 5.0 g of a condensate of 1 (or 5)-[(9Z)-9-octadecenyl] hydrogen 2-(N-tert-butoxycarbonylamino)pentanedioate and 1,3-phenylenediamine.
To 3.0 g of the thus obtained condensate was added 40 mL of methylene chloride, and following stirring of the mixture under a nitrogen atmosphere until a uniform liquid was obtained, trifluoroacetic acid (manufactured by Wako Pure Chemical Industries, Ltd.) was added until thin layer chromatography confirmed that the raw material had been completely consumed (total amount of trifluoroacetic acid added: 12.0 g). Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 2.0 g of the compound (1-6) (yield: 83%).

Elemental analysis result: Cs₂H₉₀N₄O₆
Calculated values (C: 72.01%, H: 10.46%, N: 6.46%)
Measured values (C: 72.20%, H: 10.55%, N: 6.38%)

With the exception of using the compound (1-6) as the additive for oils, sample oils were prepared in the same manner as example 1, and then evaluated in the same manner as example 1. The results are shown in Table 2.

### [Example 7] Condensate of 1 (or 5)-[(9Z)-9-octadecenyl] hydrogen 2-aminopentanedioate and ethylene glycol [Compound (1-7)]

2.5 g of 2-(N-tert-butoxycarbonylamino)pentanedioic acid (manufactured by Kanto Chemical Co., Inc.), 0.3 g of ethylene glycol (manufactured by Wako Pure Chemical Industries, Ltd.) and 1.2 g of 4-(dimethylamino)pyridine (manufactured by Wako Pure Chemical Industries, Ltd.) were dissolved in 25 mL of methylene chloride (manufactured by Wako Pure Chemical Industries, Ltd.), 2.1 g of 1-ethyl-3-(N,N'-dimethylaminopropyl)carbodiimide (manufactured by Eiweiss Chemical Corporation) was added, and the resulting mixture was reacted at room temperature for 2 hours and then at 40°C for a further 4 hours. Following reaction, to the reaction mixture were added 2.7 g of (Z)-9-octadecen-1-ol (manufactured by Wako Pure Chemical Industries, Ltd.), 1.2 g of 4-(dimethylamino)pyridine and 25 mL of methylene chloride, and following thorough stirring, an additional 2.1 g of 1-ethyl-3-(N,N'-dimethylaminopropyl)carbodiimide was added, and the resulting mixture was reacted at room temperature for 2 hours and then at 40°C for a further 4 hours. The organic layer was dried over anhydrous magnesium sulfate, and the solvent was then removed by distillation under reduced pressure at 50°C, yielding 4.9 g of a condensate of 1 (or 5)-[(9Z)-9-octadecenyl] hydrogen 2-(N-tert-butoxycarbonylamino)pentanedioate and ethylene glycol.
To 4.9 g of the thus obtained condensate was added 100 mL of methylene chloride, and following stirring of the mixture under a nitrogen atmosphere until a uniform liquid was obtained, trifluoroacetic acid (manufactured by Wako Pure Chemical Industries, Ltd.) was added until thin layer chromatography confirmed that the raw material had been completely consumed (total amount of trifluoroacetic acid added: 30.0 g). Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 2.3 g of the compound (1-7) (yield: 57%).

Elemental analysis result: C₄₈H₈₈N₂O₈
Calculated values (C: 70.20%, H: 10.80%, N: 3.41%)
Measured values (C: 70.12%, H: 10.93%, N: 3.36%)

With the exception of using the compound (1-7) as the additive for oils, sample oils were prepared in the same manner as example 1, and then evaluated in the same manner as example 2. The results are shown in Table 3.

### [Example 8] Condensate of 1 (or 5)-[(9Z)-9-octadecenyl] hydrogen 2-aminopentanedioate and 2,2-bis(4-hydroxycyclohexyl)propane [Compound (1-8)]

3.7 g of 2-(N-tert-butoxycarbonylamino)pentanedioic acid (manufactured by Kanto Chemical Co., Inc.), 1.8 g of 2,2-bis(4-hydroxycyclohexyl)propane (manufactured by Tokyo Chemical Industry Co., Ltd.) and 1.8 g of 4-(dimethylamino)pyridine (manufactured by Wako Pure Chemical Industries, Ltd.) were dissolved in 40 mL of methylene chloride (manufactured by Wako Pure Chemical Industries, Ltd.), 3.2 g of 1-ethyl-3-(N,N'-dimethylaminopropyl)carbodiimide (manufactured by Eiweiss Chemical Corporation) was added, and the resulting mixture was reacted at room temperature for 1 hour and then at 40°C for a further 8 hours. To the resulting reaction mixture were added 4.0 g of (Z)-9-octadecen-1-ol (manufactured by Wako Pure Chemical Industries, Ltd.), 3.7 g of 4-(dimethylamino)pyridine and 38 mL of methylene chloride, and following thorough stirring, an additional 6.0 g of 1-ethyl-3-(N,N'-dimethylaminopropyl)carbodiimide was added, and the resulting mixture was reacted at room temperature for 1 hour and then at 40°C for a further 10 hours. Following reaction, the reaction mixture was washed sequentially with 0.5 mol/L hydrochloric acid, a saturated aqueous solution of sodium bicarbonate, and a saturated aqueous saline solution. The organic layer was dried over anhydrous magnesium sulfate, and the solvent was then removed by distillation under reduced pressure at 50°C, yielding 8.3 g of a condensate of 1 (or 5)-[(9Z)-9-octadecenyl] hydrogen 2-(N-tert-butoxycarbonylamino)pentanedioate and 2,2-bis(4-hydroxycyclohexyl)propane.
4.0 g of the thus obtained condensate was dissolved in 60 mL of methylene chloride, and following stirring under a nitrogen atmosphere until a uniform solution was obtained, trifluoroacetic acid (manufactured by Wako Pure Chemical Industries, Ltd.) was added until thin layer chromatography confirmed that the raw material had been completely consumed (total amount of trifluoroacetic acid added: 16.0 g). Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 2.9 g of the compound (I-8) (yield: 79.1 %).

¹H-NMR (CDCl₃, δ ppm): 0.71 to 0.76 (s, 6H), 0.88 (t, 6H), 1.05 to 2.13 (m, 82H), 2.38 to 2.51 (m, 4H), 3.39 to 3.50 (m, 2H), 4.05 to 4.20 (m, 4H), 4.60 to 5.05 (m, 2H), 5.30 to 5.42 (m, 4H)

With the exception of using the compound (1-8) as the additive for oils, sample oils were prepared in the same manner as example 1, and then evaluated in the same manner as example 2. The results are shown in Table 3.

### [Example 9] Condensate of 1 (or 5)-[(9Z)-9-octadecenyl] hydrogen 2-aminopentanedioate and 4,4'-diaminodiphenyl ether [Compound (1-9)]

3.7 g of 2-(N-tert-butoxycarbonylamino)pentanedioic acid (manufactured by Kanto Chemical Co., Inc.), 1.5 g of 4,4'-diaminodiphenyl ether (manufactured by Tokyo Chemical Industry Co., Ltd.) and 1.8 g of 4-(dimethylamino)pyridine (manufactured by Wako Pure Chemical Industries, Ltd.) were dissolved in 4 mL of methylene chloride (manufactured by Wako Pure Chemical Industries, Ltd.), 3.2 g of 1-ethyl-3-(N,N'-dimethylaminopropyl)carbodiimide (manufactured by Eiweiss Chemical Corporation) was added, and the resulting mixture was reacted at room temperature for 1 hour and then at 40°C for a further 6 hours. To the resulting reaction mixture were added 4.0 g of (Z)-9-octadecen-1-ol (manufactured by Wako Pure Chemical Industries, Ltd.), 3.7 g of 4-(dimethylamino)pyridine and 40 mL of methylene chloride, and following thorough stirring, an additional 6.0 g of 1-ethyl-3-(N,N'-dimethylaminopropyl)carbodiimide was added, and the resulting mixture was reacted at room temperature for 1 hour and then at 40°C for a further 14 hours. Following reaction, the reaction mixture was washed sequentially with 0.5 mol/L hydrochloric acid, a saturated aqueous solution of sodium bicarbonate, and a saturated aqueous saline solution. The organic layer was dried over anhydrous magnesium sulfate, and the solvent was then removed by distillation under reduced pressure at 50°C, yielding 7.6 g of a crude condensate of 1 (or 5)-[(9Z)-9-octadecenyl] hydrogen 2-(N-tert-butoxycarbonylamino)pentanedioate and 4,4'-diaminodiphenyl ether. The thus obtained condensate was purified by silica gel column chromatography (developing solvent: chloroform/methanol).
4.6 g of the purified condensate was dissolved in 65 mL of methylene chloride, and following stirring under a nitrogen atmosphere until a uniform solution was obtained, trifluoroacetic acid (manufactured by Wako Pure Chemical Industries, Ltd.) was added until thin layer chromatography confirmed that the raw material had been completely consumed (total amount of trifluoroacetic acid added: 18.4 g). Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 3.2 g of the compound (1-9) (yield: 51.8%).

¹H-NMR (CDCl₃, δ ppm): 0.88 (t, 6H), 1.05 to 2.30 (m, 68H), 2.40 to 2.60 (m, 4H), 3.49 to 3.57 (m, 2H), 4.02 to 4.20 (m, 4H), 5.29 to 5.41 (m, 4H), 6.85 to 6.92 (m, 4H), 7.40 to 7.48 (m, 4H), 8.40 to 8.55 (br, 2H)

With the exception of using the compound (1-9) as the additive for oils, sample oils were prepared in the same manner as example 1. Using the methods described above for example 1, the sample oil in which the lubricant base oil A was used was evaluated for friction resistance, whereas the sample oil in which the lubricant base oil B was used was evaluated for friction resistance and wear resistance. The results are shown in Table 3.

### [Example 10] Condensate of 1 (or 5)-[(9Z)-9-octadecenyl] hydrogen 2-aminopentanedioate and bis(hydroxyethyl) disulfide [Compound (I-10)]

3.7 g of 2-(N-tert-butoxycarbonylamino)pentanedioic acid, 1.2 g of bis(hydroxyethyl) disulfide (manufactured by Tokyo Chemical Industry Co., Ltd.) and 1.8 g of 4-(dimethylamino)pyridine (manufactured by Wako Pure Chemical Industries, Ltd.) were dissolved in 40 mL of 1,2-dichloroethane (manufactured by Wako Pure Chemical Industries, Ltd.), 3.3 g of 1-ethyl-3-(N,N'-dimethylaminopropyl)carbodiimide (manufactured by Eiweiss Chemical Corporation) was added, and the resulting mixture was reacted at room temperature for 1 hour and then at 70°C for a further 6 hours. To the resulting reaction mixture were added 4.0 g of (Z)-9-octadecenyl alcohol (manufactured by Wako Pure Chemical Industries, Ltd.), 1.8 g of 4-(dimethylamino)pyridine and 40 mL of 1,2-dichloroethane, and following thorough stirring, an additional 3.3 g of 1-ethyl-3-(N,N'-dimethylaminopropyl)carbodiimide was added, and the resulting mixture was reacted at room temperature for 1 hour and then at 70°C for a further 13 hours. Following reaction, the reaction mixture was washed sequentially with 0.5 mol/L hydrochloric acid, a saturated aqueous solution of sodium bicarbonate, and a saturated aqueous saline solution. The organic layer was dried over anhydrous magnesium sulfate, and the solvent was then removed by distillation under reduced pressure at 50°C, yielding 1.6 g of a crude condensate of 1 (or 5)-[(9Z)-9-octadecenyl] hydrogen 2-(N-tert-butoxycarbonylamino)pentanedioate and bis(hydroxyethyl) disulfide.
This crude condensate was purified by silica gel column chromatography (developing solvent: n-hexane /ethyl acetate).
1.6 g of the purified condensate was dissolved in 20 mL of methylene chloride, and following stirring under a nitrogen atmosphere until a uniform solution was obtained, trifluoroacetic acid (manufactured by Wako Pure Chemical Industries, Ltd.) was added until thin layer chromatography confirmed that the raw material had been completely consumed (total amount of trifluoroacetic acid added: 4.8 g). Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 1.2 g of the compound (I-10) (yield: 27.5%).

¹H-NMR (CDCl₃, δ ppm): 0.88 (t, 6H), 1.28 (m, 44H), 1.52 (s, 6H), 1.64 (m, 4H), 1.85 to 2.50 (m, 20H), 2.93 (m, 8H), 3.44 to 3.56 (m, 2H), 3.72 (m, 1H), 4.11 (t, 4H), 4.37 (m, 8H), 5.36 (m, 4H)

With the exception of using the compound (I-10) as the additive for oils, sample oils were prepared in the same manner as example 1, and then evaluated in the same manner as example 2. The results are shown in Table 4.

### [Example 11] Condensate of 1 (or 5)-[(9Z)-9-octadecenyl] hydrogen 2-aminopentanedioate and 2,2-bis(4-hydroxyphenyl)propane [Compound (I-11)]

3.7 g of 2-(N-tert-butoxycarbonylamino)pentanedioic acid (manufactured by Kanto Chemical Co., Inc.), 1.7 g of 2,2-bis(4-hydroxyphenyl)propane (manufactured by Tokyo Chemical Industry Co., Ltd.) and 1.8 g of 4-(dimethylamino)pyridine (manufactured by Wako Pure Chemical Industries, Ltd.) were dissolved in 38 mL of methylene chloride (manufactured by Wako Pure Chemical Industries, Ltd.), and following thorough stirring, 3.2 g of 1-ethyl-3-(N,N'-dimethylaminopropyl)carbodiimide (manufactured by Eiweiss Chemical Corporation) was added, and the resulting mixture was reacted at room temperature for 1 hour and then at 40°C for a further 6 hours. To the resulting reaction mixture were added 4.0 g of (Z)-9-octadecenyl alcohol (manufactured by Wako Pure Chemical Industries, Ltd.), 3.7 g of 4-(dimethylamino)pyridine and 38 mL of methylene chloride, and following thorough stirring, an additional 6.0 g of 1-ethyl-3-(N,N'-dimethylaminopropyl)carbodiimide was added, and the resulting mixture was reacted at room temperature for 1 hour and then at 40°C for a further 10 hours. Following reaction, the reaction mixture was washed sequentially with 0.5 mol/L hydrochloric acid, a saturated aqueous solution of sodium bicarbonate, and a saturated aqueous saline solution. The organic layer was dried over anhydrous magnesium sulfate, and the solvent was then removed by distillation under reduced pressure at 50°C, yielding 8.2 g of a crude condensate of 1 (or 5)-[(9Z)-9-octadecenyl] hydrogen 2-(N-tert-butoxycarbonylamino)pentanedioate and 2,2-bis(4-hydroxyphenyl)propane.
This crude condensate was purified by silica gel column chromatography (developing solvent: hexane /ethyl acetate).
1.8 g of the purified condensate was dissolved in 25 mL of methylene chloride, and following stirring under a nitrogen atmosphere until a uniform solution was obtained, trifluoroacetic acid (manufactured by Wako Pure Chemical Industries, Ltd.) was added until thin layer chromatography confirmed that the raw material had been completely consumed (total amount of trifluoroacetic acid added: 8.1 g). Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 1.3 g of the compound (I-11) (yield: 17.9%).

¹H-NMR (CDCl₃, δ ppm): 0.87 (t, 6H), 1.05 to 1.80 (m, 52H), 1.60 (s, 6H), 1.90 to 2.08 (m, 8H), 2.18 to 2.60 (m, 8H), 4.18 (t, 4H), 4.20 to 4.27 (m, 2H), 5.28 to 5.50 (m, 4H), 6.69 to 6.76 (m, 4H), 7.05 to 7.11 (m, 4H)

With the exception of using the compound (I-11) as the additive for oils, sample oils were prepared in the same manner as example 1, and then evaluated in the same manner as example 1. The results are shown in Table 4.

### [Example 12] Condensate of 1 (or 5)-[(9Z)-9-octadecenyl] hydrogen 2-aminopentanedioate and 1,2,3-propanetriol [Compound (I-12)]

7.5 g of the 1 (or 5)-[(9Z)-9-octadecenyl] hydrogen 2-(N-tert-butoxycarbonylamino)pentanedioate (compound A) obtained in production example 1, 0.5 g of 1,2,3-propanetriol (manufactured by Tokyo Chemical Industry Co., Ltd.) and 1.8 g of 4-(dimethylamino)pyridine (manufactured by Wako Pure Chemical Industries, Ltd.) were dissolved in 40 mL of methylene chloride (manufactured by Wako Pure Chemical Industries, Ltd.), 3.3 g of 1-ethyl-3-(N,N'-dimethylaminopropyl)carbodiimide (manufactured by Eiweiss Chemical Corporation) was added, and the resulting mixture was reacted at room temperature for 1 hour and then at 40°C for a further 16 hours. The reaction mixture was washed sequentially with 0.5 mol/L hydrochloric acid, a saturated aqueous solution of sodium bicarbonate, and a saturated aqueous saline solution. The organic layer was dried over anhydrous magnesium sulfate, and the solvent was then removed by distillation under reduced pressure at 50°C, yielding 6.6 g of a crude condensate of 1 (or 5)-[(9Z)-9-octadecenyl] hydrogen 2-(N-tert-butoxycarbonylamino)pentanedioate and 1,2,3-propanetriol.
This crude condensate was purified by silica gel column chromatography (developing solvent: n-hexane /ethyl acetate).
1.2 g of the purified condensate was added in 15 mL of methylene chloride, and following stirring under a nitrogen atmosphere until a uniform solution was obtained, trifluoroacetic acid (manufactured by Wako Pure Chemical Industries, Ltd.) was added until thin layer chromatography confirmed that the raw material had been completely consumed (total amount of trifluoroacetic acid added: 4.8 g). Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 1.0 g of the compound (I-12) (yield: 15.9%).

¹H-NMR (CDCl₃, δ ppm): 0.88 (t, 9H), 1.28 (m, 66H), 1.53 (s, 6H), 1.61 (m, 6H), 1.83 to 2.46 (m, 24H), 3.49 (m, 3H), 4.06 to 4.43 (m, 11H), 5.35 (m, 6H)

With the exception of using the compound (I-12) as the additive for oils, sample oils were prepared in the same manner as example 1, and then evaluated in the same manner as example 2. The results are shown in Table 4.

### [Example 13] Condensate of 1 (or 5)-[(9Z)-9-octadecenyl] hydrogen 2-aminopentanedioate and 2-ethyl-2-(hydroxymethyl)propan-1,3-diol [Compound (I-13)]

4.5 g of the 1 (or 5)-[(9Z)-9-octadecenyl] hydrogen 2-(N-tert-butoxycarbonylamino)pentanedioate (compound A) obtained in production example 1, 0.4 g of 2-ethyl-2-(hydroxymethyl)propan-1,3-diol (manufactured by Tokyo Chemical Industry Co., Ltd.) and 1.1 g of 4-(dimethylamino)pyridine (manufactured by Wako Pure Chemical Industries, Ltd.) were dissolved in 40 mL of methylene chloride (manufactured by Wako Pure Chemical Industries, Ltd.), 1.9 g of 1-ethyl-3-(N,N'-dimethylaminopropyl)carbodiimide (manufactured by Eiweiss Chemical Corporation) was added, and the resulting mixture was reacted at room temperature for 1 hour and then at 40°C for a further 12 hours. Following reaction, the reaction mixture was washed sequentially with 0.5 mol/L hydrochloric acid, a saturated aqueous solution of sodium bicarbonate, and a saturated aqueous saline solution. The organic layer was dried over anhydrous magnesium sulfate, and the solvent was then removed by distillation under reduced pressure at 50°C, yielding 3.6 g of a crude condensate of 1 (or 5)-[(9Z)-9-octadecenyl] hydrogen 2-(N-tert-butoxycarbonylamino)pentanedioate and 2-ethyl-2-(hydroxymethyl)propan-1,3-diol.
This crude condensate was purified by silica gel column chromatography (developing solvent: n-hexane /ethyl acetate).
2.3 g of the purified condensate was added in 30 mL of methylene chloride, and following stirring under a nitrogen atmosphere until a uniform solution was obtained, trifluoroacetic acid (manufactured by Wako Pure Chemical Industries, Ltd.) was added until thin layer chromatography confirmed that the raw material had been completely consumed (total amount of trifluoroacetic acid added: 8.8 g). Following reaction, the reaction mixture was washed sequentially with distilled water, a saturated aqueous solution of sodium bicarbonate, and a saturated aqueous saline solution. The organic layer was dried over anhydrous magnesium sulfate, and the solvent was then removed by distillation under reduced pressure at 50°C, yielding 1.7 g of the compound (I-13) (yield: 15.6%).

¹H-NMR (CDCl₃, δ ppm): 0.90 (m, 12H), 1.28 (m, 68H), 1.52 (s, 6H), 1.61 (m, 6H), 1.83 to 2.45 (m, 24H), 3.49 (m, 3H), 4.08 (m, 12H), 5.35 (m, 6H)

With the exception of using the compound (I-13) as the additive for oils, sample oils were prepared in the same manner as example 1, and then evaluated in the same manner as example 2. The results are shown in Table 4.

### [Example 14] Mixed ester of 1 (or 5)-octadecyl hydrogen 2-aminopentanedioate and pentaerythritol [Compound (I-14)]

A mixture of 2.9 g of L-glutamic acid (manufactured by Tokyo Chemical Industry Co., Ltd.) and 5.4 g of stearyl alcohol (manufactured by Tokyo Chemical Industry Co., Ltd.) was heated to 80°C, and following melting of the stearyl alcohol, 2.3 g of methanesulfonic acid (manufactured by Kanto Chemical Co., Inc.) was added, and the resulting mixture was reacted at 120°C for 3 hours. Subsequently, 0.7 g of pentaerythritol (manufactured by Mitsubishi Gas Chemical Co., Inc.) was added, and reaction was continued at 120°C for 4 hours. To 2.5 g of the contents of the reaction vessel were added 50 mL of methylene chloride and 50 mL of a 2% aqueous solution of sodium hydroxide, and following phase separation, the organic layer was washed sequentially with distilled water and a saturated aqueous saline solution. Subsequently, the organic layer was dried over anhydrous magnesium sulfate, and the solvent was removed by distillation under reduced pressure at 50°C, yielding 1.8 g of the compound (I-14) (yield: 96%).

¹H-NMR (CDCl₃, δ ppm): 0.88 (m, 12H), 1.26 (m, 120H), 1.62 (m, 16H), 1.89 to 2.54 (m, 8H), 2.20 to 2.54 (m, 8H), 3.37 to 3.48 (m, 4H), 4.05 to 4.25 (m, 16H)

Using the method described in example 1, the compound (I-14) was added to the lubricant base oil A to prepare a sample oil. The friction resistance of the sample oil was evaluated using the method described in example 1. The results are shown in Table 5.

### [Example 15] Mixed ester of 1 (or 5)-octadecyl hydrogen 2-aminopentanedioate and dipentaerythritol [Compound (I-15)]

3.0 g of L-glutamic acid (manufactured by Tokyo Chemical Industry Co., Ltd.) and 5.7 g of stearyl alcohol (manufactured by Tokyo Chemical Industry Co., Ltd.) were heated to 80°C, and once the stearyl alcohol had melted, 2.4 g of methanesulfonic acid (manufactured by Kanto Chemical Co., Inc.) was added, and the resulting mixture was reacted at 120°C for 3 hours. Subsequently, 0.7 g of dipentaerythritol (manufactured by Koei Chemical Co., Ltd.) was added, and reaction was continued at 120°C for 12 hours. To 2.7 g of the contents of the reaction vessel were added 50 mL of methylene chloride and 50 mL of a 2% aqueous solution of sodium hydroxide, and following phase separation, the organic layer was washed sequentially with distilled water and a saturated aqueous saline solution. The organic layer was then dried over anhydrous magnesium sulfate, and the solvent was removed by distillation under reduced pressure at 50°C, yielding 1.8 g of the compound (I-15) (yield: 92%).

¹H-NMR (CDCl₃, δ ppm): 0.88 (m, 18H), 1.26 (m, 180H), 1.62 (m, 24H), 1.79 to 2.12 (m, 12H), 2.20 to 2.54 (m, 12H), 3.37 to 3.48 (m, 6H), 4.05 to 4.25 (m, 24H)

With the exception of using the compound (I-15) as the additive for oils, a sample oil was prepared in the same manner as example 14, and then evaluated in the same manner as example 14. The results are shown in Table 5.

### (Production example 2) 2-phthalimidopentanedioic anhydride (Compound B)

73.6 g of L-glutamic acid (manufactured by Tokyo Chemical Industry Co., Ltd.) and 74.1 g of phthalic anhydride (manufactured by Tokyo Chemical Industry Co., Ltd.) were reacted at 140°C for 2 hours. The reaction mixture was then cooled to 100°C, 102.1 g of acetic anhydride (manufactured by Wako Pure Chemical Industries, Ltd.) was added, and reaction was conducted at 100°C for 10 minutes. Subsequently, 200 mL of xylene (manufactured by Wako Pure Chemical Industries, Ltd.) was added, and the reaction mixture was cooled to room temperature. The precipitated crystals were collected by filtration, and the crystals were then washed with 20 mL of xylene and dried at 70°C, yielding 70.8 g of 2-phthalimidopentanedioic anhydride (yield: 55%).

¹H-NMR (acetone-d₆, δ ppm): 2.34 (m, 1H), 2.85 (m, 1H), 3.16 (dt, 1H), 3.27 (dt, 1H), 5.46 (dd, 1H), 7.95 (m, 4H)

### [Example 16] Condensate of 1 (or 5)-octadecyl hydrogen 2-phthalimidopentanedioate and 1,2,3-propanetriol [Compound (I-16)]

4.7 g of 2-phthalimidopentanedioic anhydride (the compound B) and 4.9 g of stearyl alcohol (manufactured by Tokyo Chemical Industry Co., Ltd.) was reacted at 120°C for 4 hours. Subsequently, 0.8 g of 1,2,3-propanetriol (manufactured by Tokyo Chemical Industry Co., Ltd.) and 0.3 g of methanesulfonic acid (manufactured by Kanto Chemical Co., Inc.) were added to the reaction product, and the resulting mixture was reacted at 120°C for 4 hours. 100 mL of methylene chloride was added, and the mixture was washed with distilled water and a saturated aqueous saline solution. The organic layer was then dried over anhydrous magnesium sulfate, the solvent was removed by distillation under reduced pressure at 50°C, and the crude product was purified by silica gel column chromatography (developing solvent: hexane / ethyl acetate), yielding 7.3 g of the compound (I-16) (yield: 72%).

¹H-NMR (CDCl₃, δ ppm): 0.88 (m, 6H), 1.26 (m, 60H), 1.56 (m, 4H), 2.36 to 2.67 (m, 8H), 3.96 to 2.18 (m, 8H), 4.23 (br, 1H), 4.92 (m, 2H), 7.73 to 7.88 (m, 8H)

With the exception of using the compound (I-16) as the additive for oils, sample oils were prepared in the same manner as example 1, and the friction resistance of these sample oils was then evaluated in the same manner as example 1. The results are shown in Table 5.

### [Example 17] Condensate of 1 (or 5)-octadecyl hydrogen 2-phthalimidopentanedioate and 2-ethyl-2-(hydroxymethyl)propan-1,3-diol [Compound (I-17)]

1.2 g of 2-phthalimidopentanedioic anhydride (the compound B) and 1.2 g of stearyl alcohol (manufactured by Tokyo Chemical Industry Co., Ltd.) were reacted at 120°C for 3 hours. Subsequently, 0.2 g of 2-ethyl-2-(hydroxymethyl)propan-1,3-diol (manufactured by Tokyo Chemical Industry Co., Ltd.) and 0.1 g of methanesulfonic acid (manufactured by Kanto Chemical Co., Inc.) were added to the reaction product, and the resulting mixture was reacted at 120°C for 3 hours. 40 mL of methylene chloride was added, and the mixture was washed sequentially with distilled water and a saturated aqueous saline solution. The organic layer was then dried over anhydrous magnesium sulfate, the solvent was removed by distillation under reduced pressure at 50°C, and the crude product was purified by silica gel column chromatography (developing solvent:
hexane / ethyl acetate), yielding 2.4 g of the compound (I-17) (yield: 95%).

¹H-NMR (CDCl₃, δ ppm): 0.70 (m, 3H), 0.88 (m, 9H), 1.26 (m, 92H), 1.56 (m, 6H), 2.34 to 2.67 (m, 12H), 3.80 to 4.13 (m, 12H), 4.91 (m, 3H), 7.76 to 7.88 (m, 12H)

With the exception of using the compound (I-17) as the additive for oils, sample oils were prepared in the same manner as example 1, and then evaluated in the same manner as example 1. The results are shown in Table 6.

### [Example 18] Condensate of 1 (or 5)-octadecyl hydrogen 2-phthalimidopentanedioate and pentaerythritol [Compound (1-18)]

10.4 g of the 2-phthalimidopentanedioic anhydride (the compound B) obtained in production example 2 and 10.8 g of stearyl alcohol (manufactured by Tokyo Chemical Industry Co., Ltd.) were reacted at 120°C for 2 hours. The reaction product was purified by silica gel column chromatography (developing solvent: n-hexane / ethyl acetate), yielding 12.7 g of 1 (or 5)-octadecyl hydrogen 2-phthalimidopentanedioate (yield: 59.1%).
¹H-NMR (CDCl₃, δ ppm): 0.88 (t, 3H), 1.26 (m, 30H), 1.56 (m, 2H), 2.38 (m, 4H), 2.48 to 2.65 (m, 2H), 3.99 (t, 2H), 5.00 (m, 1H), 7.76 (m, 2H), 7.87 (m, 2H)

Subsequently, 10.6 g of the thus obtained 1 (or 5)-octadecyl hydrogen 2-phthalimidopentanedioate, 0.7 g of pentaerythritol (manufactured by Wako Pure Chemical Industries, Ltd.) and 2.4 g of 4-(dimethylamino)pyridine (manufactured by Wako Pure Chemical Industries, Ltd.) were dissolved in 60 mL of methylene chloride (manufactured by Wako Pure Chemical Industries, Ltd.), 4.2 g of 1-ethyl-3-(N,N'-dimethylaminopropyl)carbodiimide (manufactured by Eiweiss Chemical Corporation) was added, and the resulting mixture was reacted at room temperature for 1 hour and then at 40°C for a further 4 hours. Following reaction, the reaction mixture was washed sequentially with 0.5 mol/L hydrochloric acid, a saturated aqueous solution of sodium bicarbonate, and a saturated aqueous saline solution. The organic layer was dried over anhydrous magnesium sulfate, and the solvent was then removed by distillation under reduced pressure at 50°C, yielding 10.4 g of a crude condensate of 1 (or 5)-monooctadecyl hydrogen 2-phthalimidopentanedioate and pentaerythritol.

This crude condensate was purified by silica gel column chromatography (developing solvent: n-hexane / ethyl acetate), yielding 8.4 g of the compound (I-18) (yield: 80.6%).
¹H-NMR (CDCl₃, δ ppm): 0.88 (t, 12H), 1.25 (m, 120H), 1.55 (m, 8H), 2.32 to 2.50 (m, 16H), 3.69 to 3.99 (m, 16H), 4.89 (m, 4H), 7.73 (m, 8H), 7.85 (m, 8H)

With the exception of using the compound (I-18) as the additive for oils, a sample oil was prepared in the same manner as example 14, and then evaluated in the same manner as example 14. The results are shown in Table 6.

### [Example 19] Condensate of 1 (or 5)-octadecyl hydrogen 2-phthalimidopentanedioate and dipentaerythritol [Compound (I-19)]

In a 50 mL reaction flask, 7.8 g of 2-phthalimidopentanedioic anhydride (the compound B) and 8.1 g of stearyl alcohol (manufactured by Tokyo Chemical Industry Co., Ltd.) were reacted at 120°C for 3 hours. Subsequently, 1.3 g of dipentaerythritol (manufactured by Koei Chemical Co., Ltd.) and 0.1 g of methanesulfonic acid (manufactured by Kanto Chemical Co., Inc.) were added over a period of 5 minutes, and the resulting mixture was reacted under a nitrogen atmosphere at 120°C for 4 hours.
Following reaction, the reaction mixture was cooled to 40°C, 75 mL of methylene chloride was added, and the mixture was washed with water. The organic layer was then dried over anhydrous magnesium sulfate, and the solvent was removed by distillation. The resulting crude product was purified by silica gel column chromatography (developing solvent: hexane / ethyl acetate), yielding 12.9 g of the compound (I-19) (yield: 77%).

¹H-NMR (CDCl₃, δ ppm): 0.88 (t, 18H), 1.26 (m, 180H), 1.57 (m, 12H), 2.40 (m, 12H), 2.52 (m, 6H), 2.63 (m, 6H), 3.20 (br, 4H), 4.00 (m, 12H), 4.13 (m, 12H), 5.00 (dd, 6H), 7.76 (m, 12H), 7.88 (m, 12H)

With the exception of using the compound (I-19) as the additive for oils, sample oils were prepared in the same manner as example 1, and then evaluated in the same manner as example 1. The results are shown in Table 6.

### (Comparative example 1)

With the exception of using dibenzyl disulfide (DBDS) manufactured by Tokyo Chemical Industry Co., Ltd. as the additive for oils, sample oils were prepared in the same manner as example 1, and then evaluated in the same manner as example 1. The results are shown in Table 7. In the tables, "N.T." indicates "not tested".

**[Table 1]**

| | Example 1 | | Example 2 | | Example 3 | |
|---|---|---|---|---|---|---|
| Additive for oils | (I-1) | | (1-2) | | (1-3) | |
| Lubricant base oil | A | B | A | B | A | B |
| Coefficient of kinetic friction (40°C) | 0.095 | 0.093 | 0.154 | N.T. | 0.104 | 0.127 |
| Coefficient of kinetic friction (80°C) | 0.102 | 0.149 | 0.175 | N.T. | 0.095 | 0.144 |
| Coefficient of kinetic friction (120°C) | 0.106 | 0.192 | 0.196 | N.T. | 0.075 | 0.138 |
| Coefficient of kinetic friction (150°C) | 0.093 | 0.214 | 0.175 | N.T. | 0.071 | 0.141 |
| Wear scar diameter (mm) | 0.42 | 0.77 | 0.65 | 0.79 | 0.69 | 0.76 |

**[Table 2]**

| | Example 4 | | Example 5 | | Example 6 | |
|---|---|---|---|---|---|---|
| Additive for oils | (I-4) | | (I-5) | | (I-6) | |
| Lubricant base oil | A | B | A | B | A | B |
| Coefficient of kinetic friction (40°C) | 0.131 | 0.106 | 0.177 | 0.145 | 0.212 | 0.107 |
| Coefficient of kinetic friction (80°C) | 0.154 | 0.107 | 0.178 | 0.132 | 0.182 | 0.140 |
| Coefficient of kinetic friction (120°C) | 0.103 | 0.118 | 0.187 | 0.153 | 0.194 | 0.150 |
| Coefficient of kinetic friction (150°C) | 0.100 | 0.109 | 0.198 | 0.166 | 0.204 | 0.172 |
| Wear scar diameter (mm) | 0.66 | 0.69 | 0.52 | 0.77 | 0.36 | 0.80 |

**[Table 3]**

| | Example 7 | | Example 8 | | Example 9 | |
|---|---|---|---|---|---|---|
| Additive for oils | (I-7) | | (I-8) | | (I-9) | |
| Lubricant base oil | A | B | A | B | A | B |
| Coefficient of kinetic friction (40°C) | 0.085 | N.T. | 0.121 | N.T. | 0.167 | 0.120 |
| Coefficient of kinetic friction (80°C) | 0.139 | N.T. | 0.140 | N.T. | 0.138 | 0.149 |
| Coefficient of kinetic friction (120°C) | 0.199 | N.T. | 0.172 | N.T. | 0.105 | 0.167 |
| Coefficient of kinetic friction (150°C) | 0.227 | N.T. | 0.189 | N.T. | 0.146 | 0.169 |
| Wear scar diameter (mm) | 0.58 | 0.80 | 0.52 | 0.71 | N.T. | 0.73 |

**[Table 4]**

| | Example 10 | | Example 11 | | Example 12 | | Example 13 | |
|---|---|---|---|---|---|---|---|---|
| Additive for oils | (I-10) | | (I-11) | | (I-12) | | (I-13) | |
| Lubricant base oil | A | B | A | B | A | B | A | B |
| Coefficient of kinetic friction (40°C) | 0.097 | N.T. | 0.100 | 0.133 | 0.106 | N.T. | 0.124 | N.T. |
| Coefficient of kinetic friction (80°C) | 0.091 | N.T. | 0.112 | 0.186 | 0.105 | N.T. | 0.103 | N.T. |
| Coefficient of kinetic friction (120°C) | 0.180 | N.T. | 0.101 | 0.192 | 0.118 | N.T. | 0.107 | N.T. |
| Coefficient of kinetic friction (150°C) | 0.149 | N.T. | 0.165 | 0.171 | 0.138 | N.T. | 0.115 | N.T. |
| Wear scar diameter (mm) | 0.61 | 0.57 | 0.53 | 0.73 | 0.56 | 0.77 | 0.52 | 0.78 |

**[Table 5]**

| | Example 14 | Example 15 | Example 16 | |
|---|---|---|---|---|
| Additive for oils | (I-14) | (I-15) | (I-16) | |
| Lubricant base oil | A | A | A | B |
| Coefficient of kinetic friction (40°C) | 0.088 | 0.090 | 0.106 | 0.120 |
| Coefficient of kinetic friction (80°C) | 0.098 | 0.098 | 0.148 | 0.137 |
| Coefficient of kinetic friction (120°C) | 0.197 | 0.173 | 0.160 | 0.183 |
| Coefficient of kinetic friction (150°C) | 0.187 | 0.165 | 0.150 | 0.184 |

**[Table 6]**

| | Example 17 | | Example 18 | Example 19 | |
|---|---|---|---|---|---|
| Additive for oils | (I-17) | | (I-18) | (I-19) | |
| Lubricant base oil | A | B | A | A | B |
| Coefficient of kinetic friction (40°C) | 0.119 | 0.126 | 0.085 | 0.099 | 0.102 |
| Coefficient of kinetic friction (80°C) | 0.089 | 0.163 | 0.091 | 0.101 | 0.100 |
| Coefficient of kinetic friction (120°C) | 0.150 | 0.201 | 0.100 | 0.115 | 0.106 |
| Coefficient of kinetic friction (150°C) | 0.130 | 0.209 | 0.112 | 0.135 | 0.122 |
| Wear scar diameter (mm) | 0.54 | 0.79 | N.T. | 0.55 | 0.79 |

**[Table 7]**

| | Comparative example 1 | |
|---|---|---|
| Additive for oils | DBDS | |
| Lubricant base oil | A | B |
| Coefficient of kinetic friction (40°C) | 0.419 | 0.177 |
| Coefficient of kinetic friction (80°C) | 0.432 | 0.208 |
| Coefficient of kinetic friction (120°C) | 0.410 | 0.209 |
| Coefficient of kinetic friction (150°C) | 0.389 | 0.215 |
| Wear scar diameter (mm) | 0.77 | 0.93 |

In Tables 1 to 7, smaller values for the coefficient of kinetic friction indicate superior friction resistance properties for the sample oil, and smaller values for the wear scar diameter indicate superior wear resistance properties for the sample oil. By using the compound (I) as an additive for oils, sample oils having superior levels of friction resistance and wear resistance were able to be provided.

The present invention is able to provide an additive for oils comprising a compound that is capable of imparting oils such as lubricant base oils or fuel oils with superior wear resistance properties or superior friction resistance properties.

## Claims

1. An additive for oils, comprising: a compound represented by formula (I),
[Chemical Formula 1] **(A)ₘ-W-(B)ₙ** (I)
wherein
m represents an integer of 0 to 4,
n represents an integer of 2 to 6,
m+n represents an integer of 2 to 6,
W is a group of valency (m+n) generated by removing (m+n) hydrogen atoms on carbon atoms from a compound selected from the group consisting of hydrocarbons of 2 to 20 carbon atoms, ethers of 4 to 20 carbon atoms, amines of 3 to 20 carbon atoms, sulfides of 4 to 20 carbon atoms and disulfides of 4 to 20 carbon atoms,
A represents hydroxy or amino,
when m is 2 or greater, As may be identical or different, and
Bs may be identical or different, and
B represents formula (II), 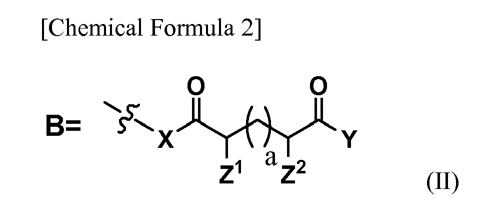 wherein
"a"s are identical or different, and each represents 0 or 1,
Xs are identical or different, and each represents an oxygen atom or NH,
Ys are identical or different, and each represents OR¹ or NHR², wherein R¹ and R² are identical or different, and each represents alkyl optionally having one or more substituents, alkenyl optionally having one or more substituents, aryl optionally having one or more substituents, aralkyl optionally having one or more substituents, cycloalkyl optionally having one or more substituents or cycloalkenyl optionally having one or more substituents,
Z¹s may be identical or different,
Z²s may be identical or different, and
within a single B, one of Z¹ and Z² represents a hydrogen atom, and another represents formula (III),
[Chemical Formula 3] **-NR³R⁴** (III)
wherein R³ and R⁴ are identical or different, and each represents a hydrogen atom, alkyl optionally having one or more substituents, alkenyl optionally having one or more substituents, aryl optionally having one or more substituents, aralkyl optionally having one or more substituents, cycloalkyl optionally having one or more substituents, cycloalkenyl optionally having one or more substituents, alkanoyl optionally having one or more substituents, alkenoyl optionally having one or more substituents, aroyl optionally having one or more substituents, cycloalkylcarbonyl optionally having one or more substituents, alkyloxycarbonyl optionally having one or more substituents, alkenyloxycarbonyl optionally having one or more substituents, aryloxycarbonyl optionally having one or more substituents or cycloalkyloxycarbonyl optionally having one or more substituents, or R³ and R⁴ form a nitrogen-containing heterocyclic group optionally having one or more substituents in combination with an adjacent nitrogen atom thereto,
formula (IV),
[Chemical Formula 4] **-N=CR⁵R⁶** (IV)
wherein R⁵ and R⁶ are identical or different, and each represents a hydrogen atom, alkyl optionally having one or more substituents or alkenyl optionally having one or more substituents, or R⁵ and R⁶ form cycloalkylidene optionally having one or more substituents in combination with an adjacent carbon atom thereto, or
formula (V), 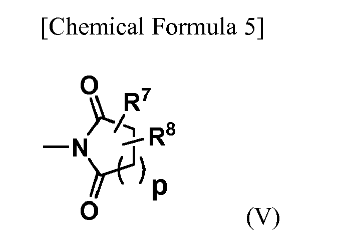 wherein p represents an integer of 1 to 3, R⁷ and R⁸ are identical or different, and each represents a hydrogen atom, alkyl optionally having one or more substituents, alkenyl optionally having one or more substituents, aryl optionally having one or more substituents, aralkyl optionally having one or more substituents, cycloalkenyl optionally having one or more substituents, alkanoyl optionally having one or more substituents, alkenoyl optionally having one or more substituents, aroyl optionally having one or more substituents or cycloalkylcarbonyl optionally having one or more substituents, or R⁷ and R⁸ form, in combination with two carbon atoms adjacent thereto, cycloalkane optionally having one or more substituents or an aromatic ring optionally having one or more substituents.

2. The additive for oils according to claim 1, wherein "a"s are identical, and each is 1.

3. The additive for oils according to claim 1 or claim 2, wherein
n is an integer of 2 to 4, m is an integer of 0 to 2, m+n is an integer of 2 to 4,
W is a group of valency (m+n) generated by removing (m+n) hydrogen atoms on carbon atoms from hydrocarbon, and
said hydrocarbon is alkane of 2 to 10 carbon atoms.

4. The additive for oils according to claim 1 or claim 2, wherein
n is 2, m is 0, and
W is formula (VI), 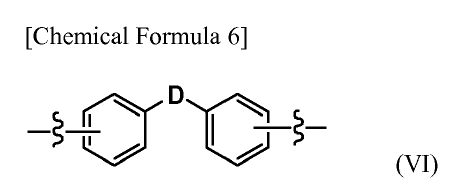 wherein D represents an oxygen atom, a sulfur atom, methylene or dimethylmethylene.

5. The additive for oils according to claim 1 or claim 2, wherein
n is 2, m is 0, and
W is formula (VII), 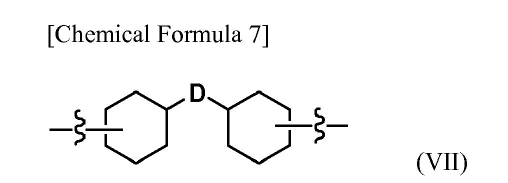 wherein D represents an oxygen atom, a sulfur atom, methylene or dimethylmethylene.

6. The additive for oils according to claim 1 or claim 2, wherein
n is 2, m is 0, and
W is formula (VIII), 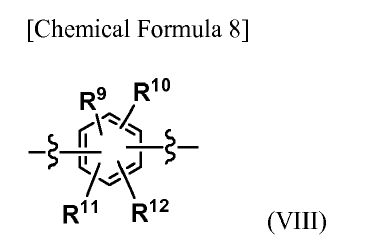 wherein R⁹, R¹⁰, R¹¹ and R¹² are identical or different, and each represents a hydrogen atom, methyl or ethyl.

7. The additive for oils according to claim 1 or claim 2, wherein
n is 2, m is 0, and
W is formula (IX), 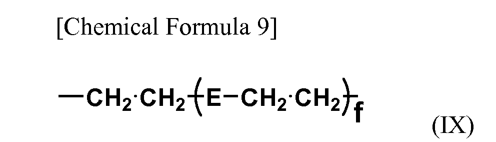 wherein f represents an integer of 1 to 4, and E represents an oxygen atom or formula (X), 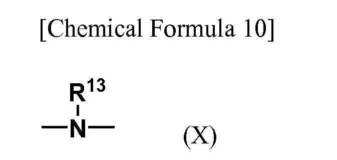 wherein R¹³ represents a hydrogen atom, methyl or ethyl.

8. The additive for oils according to claim 1 or claim 2, wherein
n is 2, m is 0, and
W is formula (XI), 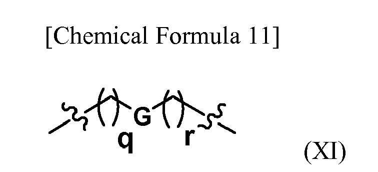 wherein G represents -S- or -S-S-, and q and r are identical or different and each represents 2 or 3.

9. The additive for oils according to claim 8, wherein q is 2 and r is 2.

10. The additive for oils according to claim 1 or claim 2, wherein
n is 2 or 3, m is 0 or 1, m+n is 3, and
W is formula (XII).

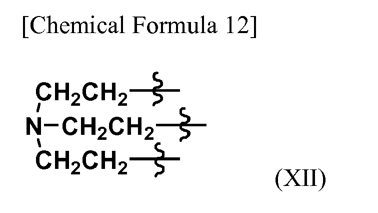

11. The additive for oils according to claim 1 or claim 2, wherein
n is 2 or 3, m is 0 or 1, m+n is 3, and
W is formula (XIII).

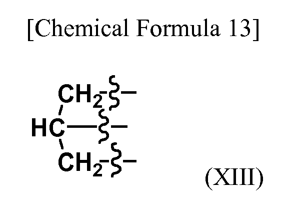

12. The additive for oils according to claim 1 or claim 2, wherein
n is 2 or 3, m is 0 or 1, m+n is 3, and
W is formula (XIV), 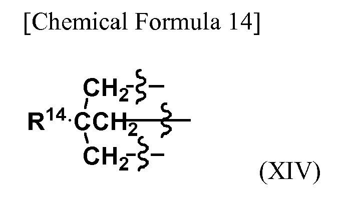 wherein R¹⁴ represents a hydrogen atom, alkyl of 1 to 6 carbon atoms, or alkenyl of 1 to 6 carbon atoms.

13. The additive for oils according to claim 12, wherein R¹⁴ is ethyl.

14. The additive for oils according to claim 1 or claim 2, wherein
n is 2 or 3, m is 0 or 1, m+n is 3, and
W is formula (XV).

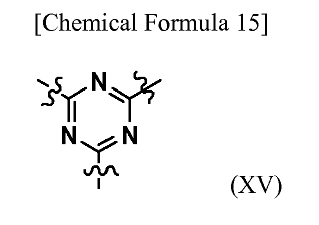

15. The additive for oils according to claim 1 or claim 2, wherein
n is an integer of 2 to 4, m is an integer of 0 to 2, m+n is 4, and
W is formula (XVI).

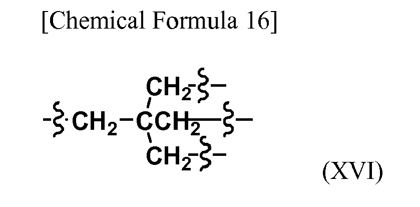

16. The additive for oils according to claim 1 or claim 2, wherein
n is an integer of 2 to 6, m is an integer of 0 to 4, m+n is 6, and
W is formula (XVII).

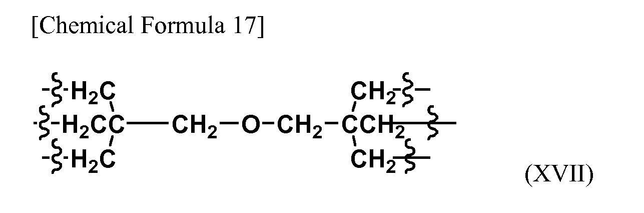

17. The additive for oils according to any one of claims 1 to 16, wherein
within a single B, one of Z¹ and Z² is a hydrogen atom, and another is formula (III).

18. The additive for oils according to any one of claims 1 to 16, wherein
within a single B, one of Z¹ and Z² is a hydrogen atom, and another is formula (V).

19. The additive for oils according to claim 18, wherein formula (V) is phthalimido.

20. The additive for oils according to any one of claims 1 to 19, wherein Xs are identical, and each is an oxygen atom.

21. The additive for oils according to any one of claims 1 to 19, wherein Xs are identical, and each is NH.

22. The additive for oils according to any one of claims 3 to 21, wherein Ys are identical, and each is OR', wherein R¹ is as defined above.

23. The additive for oils according to claim 22, wherein R¹ is alkyl optionally having one or more substituents or alkenyl optionally having one or more substituents.

24. The additive for oils according to any one of claims 1 to 3 and claims 10 to 23, wherein m is 0.

25. A lubricant, comprising: the additive for oils according to any one of claims 1 to 24, and a lubricant base oil.

26. The lubricant according to claim 25, wherein
the lubricant base oil is at least one material selected from the group consisting of mineral oils, poly-α-olefins, fatty acid esters, polyalkylene glycols, phosphates, silicones, silicates, polyphenyl ethers, alkylbenzenes, synthetic naphthenes, gas-to-liquid (GTL) products, and vegetable oils.

27. An ester compound represented by formula (Ia),
[Chemical Formula 18] **(HO)ₘₐ-W^{a}-(B^{a})ₙₐ** (Ia)
wherein
ma represents an integer of 0 to 2,
na represents an integer of 2 to 4,
ma+na represents an integer of 2 to 4,
W^{a} is a group of valency (ma+na) generated by removing (ma+na) hydrogen atoms on carbon atoms from alkane of 2 to 10 carbon atoms, and
B^{a}s may be identical or different,
B^{a} represents a group represented by formula (IIa), 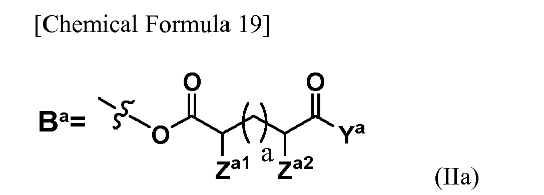 wherein
"a"s are identical or different, and each represents 0 or 1,
Y^{a}s are identical or different, and each represents -OR^{1a}, wherein R^{1a} represents alkyl of 1 to 20 carbon atoms optionally having one or more substituents, or alkenyl of 1 to 20 carbon atoms optionally having one or more substituents,
Z^{a1}s may be identical or different,
Z^{a2}s may be identical or different, and
within a single B^{a}, one of Z^{1a} and Z^{2a} represents a hydrogen atom, and another represents phthalimido.

28. The ester compound according to claim 27, wherein ma is 0.

29. The ester compound according to claim 27 or claim 28, wherein
R^{1a} is alkyl of 12 to 20 carbon atoms optionally having one or more substituents, or alkenyl of 12 to 20 carbon atoms optionally having one or more substituents.
